# EUROPEAN PATENT SPECIFICATION

(11) **EP 0 613 496 B1**
(45) Date of publication and mention of the grant of the patent: **20.01.1999**
(21) Application number: 92925133.8
(22) Date of filing: 12.11.1992
(51) Int. Cl.: C07K 14/78, C12N 15/11, C12N 15/62, C12P 21/02, A61M 31/00, A61K 38/39, A61L 27/00, D01F 4/00

(54) **COLLAGEN-LIKE POLYPEPTIDES**
KOLLAGENARTIGE POLYPEPTIDE
POLYPEPTIDES ANALOGUES AU COLLAGENE

(30) Priority: 12.11.1991 US 791743
(43) Date of publication of application: 07.09.1994
(73) Proprietor: E.I. DU PONT DE NEMOURS AND COMPANY, Wilmington Delaware 19898 (US)
(72) Inventor: GARDNER, Kenncorwin, Wilmington, DE 19803 (US); LOCK, Robert, Lee, Newark, DE 19711 (US); O'BRIEN, John, Philip, Blue Springs, MO 64015-2747 (US); SALEMME, Francis, Raymond, Kennett Square, PA 19348 (US)
(74) Representative: Jones, Alan John
(86) International application number: US9209655
(87) International publication number: WO9310231

(56) References cited:
- EP-A- 0 196 197
- WO-A-88/05082
- WO-A-90/05177
- WO-A-91/07496
- TIBTECH vol. 8, November 1990, pages 309 - 311 CAPPELLO, J. 'The biological production of protein polymers and their use'

## Description

### FIELD OF THE INVENTION

This invention relates to novel collagen-like polypeptides. More specifically, it relates to collagen-like polymers which incorporate functional segments conferring chemical or biological activity and structural elements designed to enhance processability.

### BACKGROUND OF THE INVENTION

Collagen is the most important structural protein in the bodies of higher animals, accounting for about a third of the total protein mass in most vertebrates. The prevalence of collagen in the human body makes collagen-like constructs interesting as candidates for a wide range of biomedical applications, especially as implants to replace or augment damaged or diseased structural tissues.

Collagen may be isolated from the skin, tendon, or bone of vertebrates and is also found throughout the invertebrates. Naturally derived collagen materials have a wide range of chemical structures, depending on their source. These materials may be altered to provide new materials, as for example, leather and gelatin.

Collagen shows several levels of structural hierarchy. The individual peptide chains are 1000 - 1100 amino acids long and have amino acid sequences consisting of repeating triplets

Gly - x - y,

where x and y can be any amino acids but are most likely to be proline or hydroxyproline. The 20 basic amino acids and hydroxyproline with their corresponding one and three-letter symbols are listed in Table 12. Proline is incorporated into peptide chains during protein synthesis; hydroxyproline is not incorporated during synthesis but instead is derived from proline by post-synthesis modification. Any proline residue in natural collagen or in any of the collagen-like polymers which are the subject of this invention can in principle be converted to hydroxyproline by appropriate chemical or enzymatic treatment. Therefore, any reference to proline in the polymer structure or composition of matter of any final product should be understood, by implication, as a reference to proline or hydroxyproline. However, any reference to proline in any structure as initially synthesized in vivo, or to a DNA sequence which codes for proline, should be understood as a reference to proline only, since hydroxyproline cannot be incorporated during protein synthesis, and there is no DNA sequence which codes for hydroxyproline.

The frequent Pro (and Hyp) residues direct the peptide chain into a (left-handed) helical conformation, one turn per triplet. The occurrence of Gly (no bulky side chain) at every third position allows three helical chains to pack together into a supercoiled, right-handed triple helix and it is this triple helix, with 30-40 residues per turn, which is considered the characteristic, defining structural feature of collagen. Chemical cross-links are formed between the three peptide chains to stabilize the helix.

The rod-like triple helix structure is referred to as a tropocollagen molecule. Tropocollagen molecules align head to tail in parallel bundles to form collagen fibrils. Head to tail gaps between tropocollagen molecules are staggered in a regular way along the length of each fibril to produce a characteristic pattern of striations. Collagen fibrils may be organized into cross-linked, parallel bundles to form fibers, as in tendons, or may exist as randomly interlaced, cross-linked networks, as in skin.

The ability to clone specific DNA sequences in bacteria has opened the potential for the production of novel proteins by bacteria. These techniques allow the scientist to design constructs with a wider range of properties than are available in naturally derived proteins.

Ferrari, et al., PCT published application WO 88/03533, discloses methods for the production of high molecular weight peptides containing repeating sequences. Nucleic acid sequences are built up by synthesizing nucleic acid oligomers which express a plurality of individual repetitive peptide units and the oligomers are joined to provide a polynucleotide of the desired length. The individual units coding for the oligomeric peptide sequences utilize amino acid codon redundancy to avoid problems previously associated with genes containing multiple repeating units. The individual peptide units have from 4 to 30 amino acids, usually having the same amino acid appear at least twice in the same unit, generally separated by at least one amino acid.

Cappello & Ferrari, PCT published application WO 90/05177, discloses polypeptide polymers prepared by recombinant techniques which are based on naturally occurring proteins such as silk and collagen, which are modified by introduction of an intervening amino-acid sequence which is capable of interacting with molecules in the environment and which generally does not participate in the aligned elements of the polymers. The intervening sequence may be a naturally occurring sequence or a modified naturally occurring sequence and may provide chemically active sites for cross-linking or to bind antibodies or other molecules. Collagen-like polymers (CLP) designed to undergo thermoreversible gelation at high temperatures are discussed. The CLP polymers are comprised predominantly of the repeating tripeptide sequence, GPP, with other tripeptides included to decrease the overall repetitiveness of the gene. CLP polymers containing cell attachment functionality through the introduction of sequence, Gly-Leu-Pro-Gly-Pro-Lys-Gly-Asp-Arg-Gly-Asp-Ala-Gly-Pro-Lys-Gly-Ala-Asp-Gly-Ser-Pro, for use as soft coating materials are also discussed.

Williams, et al. published PCT application WO 88/05082, discloses processes for the microbial production of peptide oligomers including collagen analogues.

Because native collagen is cross-linked, it cannot be redissolved for spinning, shaping, etc., without chemical or enzymatic digestion and consequent loss of molecular weight. Digestion also makes it impossible to reconstitute the full hierarchy of structure found in native collagen, since some of the peptide segments that are destroyed in digestion are among the ones which direct the formation of high-level structure. Finally, native collagen is not readily modified to incorporate specific chemical or biological functionality.

Although various bioengineered collagen-like polymers have been described in the sources cited above, the structures of these polymers have not incorporated any features specifically intended to enhance processability. In particular, no attempt has been made to include structures which would provide pH-dependent control of polymer solubility to facilitate the spinning of fibers or the fabrication of other shaped articles. There have also been no attempts made to include structures designed to provide regularly-spaced sites for controlled chemical cross-linking of polymer chains, even though the presence of cross-links is a characteristic of natural collagen, and cross-linking is likely to be necessary to fix (stabilize) the secondary and tertiary structures of collagen analogs in fibers and other shaped articles.

The pH-dependent control of polymer solubility provided by structures designed to facilitate fiber spinning also facilitates chemical or enzymatic derivatization of the polymers, particularly the chemical or enzymatic conversion of proline to hydroxyproline. It is well known that the temperature at which the thermal denaturation (the breakup of the characteristic triple helix upon heating) of collagen-like polymers occurs is a function of the ratio of hydroxyproline to proline in the polymer structure. It is likely that some proline-to-hydroxyproline conversion will be required to produce materials which are conformationally stable without cross-linking at physiological temperatures. pH-dependent control of polymer solubility will facilitate this conversion.

### SUMMARY OF THE INVENTION

The present invention provides bioengineered collagen-like polypeptides having structures designed to enhance processability. These polypeptides include block copolymers of the formula:

[C_{f1} (Aⱼ₁ Bⱼ₂)ₖ C_{f2}]ₘ

where,
A = a block peptide unit having amino acid sequences of the formula, [Gly - x - y]₃₋₃₀, wherein about 50 to 75% of the amino acids, x and y, are each independently selected from the group consisting of proline and hydroxyproline, each amino acid sequence, [Gly - x - y], being the same or different;
B = a block peptide unit having amino acid sequences of the formula, [Gly - x - y]₃₋₃₀, wherein about 25 to 60% of the amino acids, x and y, are each independently selected from the group consisting of proline and hydroxyproline, each amino acid sequence, [Gly - x - y], being the same or different;
C = a block peptide unit having amino acid sequences of the formula, [Gly - x - y]₃₋₃₀, wherein about 25 to 60% of the amino acids, x and y, are each independently selected from the group consisting of proline and hydroxyproline, and at least one of the remaining amino acids, x and y, is independently selected from the group consisting of lysine, arginine, histidine, cysteine, tyrosine, aspartic acid, and glutamic acid, each amino acid sequence, [Gly - x - y], being the same or different;
   f1, k, f2, and m are each equal to or greater than one; and
   the sum of j1 and j2 is equal to or greater than one.

Collagen-like polypeptides comprising of the above-described A and B block peptide units and collagen-like polypeptides comprising of the above-described C block peptide units are also included in this invention.

Particular constructs of block unit A have sequences where all of the amino acids, x and y, which are not proline or hydroxyproline, are selected from the group consisting of glycine, alanine, and serine. For example, block unit A may be composed of the amino acid sequence,

Gly-Ala-Pro-Gly-Pro-Ala-Gly-Pro-Pro.

Particular constructs of block unit B sequences include those sequences, where at least about 66% of the amino acids, x and y, are each independently selected from the group consisting of proline, hydroxyproline, glycine, alanine, and serine.

These sequences include, for example, the following: 
Gly-Ser-Arg-Gly-Asp-Pro-Gly-Pro-Pro

Gly-Ala-Gln-Gly-Pro-Ala-Gly-Pro-Gly

Gly-Gly-Arg-Gly-Asp-Ser-Gly-Pro-Pro;
 and

Gly-Pro-Pro-Gly-Gly-Arg-Gly-Asp-Ser.

Other examples of B block units include: Gly-Tyr-Ile-Gly-Ser-Arg-Gly-Pro-Pro; and

Gly-Pro-Pro-Gly-Tyr-Ile-Gly-Ser-Arg.

C-type blocks include, for example, sequences where at least about 50% of the amino acids, x and y, are each independently selected from the group consisting of proline, hydroxyproline, glycine, alanine, and serine.

At least one of the remaining amino acids, x and y, is independently selected from the group consisting of aspartic acid, glutamic acid, lysine, arginine, histidene, tyrosine, and cysteine to provide pH-dependent ionic charge for control of solubility and/or from the group of lysine, cysteine, tyrosine, aspartic acid, and glutamic acid to provide reactive sites for derivatization or cross-linking.

Examples of amino acid sequences for C block units include: 
Gly-Ala-His-Gly-Pro-Ala-Gly-Pro-Lys.

Gly-Pro-Lys-Gly-Lys-Ala-Gly-Pro-Lys

Gly-Pro-His-Gly-Lys-Ala-Gly-Pro-Lys

Gly-Pro-His-Gly-Lys-Ala-Gly-Pro-His

Gly-Pro-His-Gly-His-Ala-Gly-Pro-His

Gly-Pro-Glu-Gly-His-Ala-Gly-Pro-Lys

Gly-Pro-Glu-Gly-His-Ala-Gly-Pro-His

Gly-Pro-Glu-Gly-His-Ala-Gly-Pro-Glu;
 and

Gly-Pro-Glu-Gly-Glu-Ala-Gly-Pro-Glu

This invention includes collagen-like block copolymers selected from the group consisting of:

[ C₂ A₂₄ C₂ ]ₙ

[ C₂ A₁₂ C₂ ]ₙ

[ C₂ (A B)₁₂ C₂ ]n

[ C₂ B₁₂ C₂ ]ₙ

[ C₂ B₆ C₂ ]ₙ;

and

[ C₂ B₂₄ C₂ ]ₙ.

Another aspect of this invention is collagen-like materials composed of the above block copolymers, including polymeric blends with natural collagen. Such materials include fibers, films, coatings, and medical implants.

### BRIEF DESCRIPTION OF THE FIGURES

Figure 1 illustrates the construction of the plasmid vector pPT 0140, which contains the two "c₂" units, which were designed to form the ends of the monomer units of the model proteins DCP-1 through DCP-6. The two C₂ units in pPT 0140 are separated by a unique BanI REN site into which additional block units were subsequently inserted.

Figure 2 illustrates the construction of the DCP-1, DCP-2, and DCP-3 monomer units by the insertion of multimerized a₂ units (DCP-1 and DCP-2) or (ab)₂ units (DCP-3) into the pPT 0140 vector.

Figure 3 illustrates the construction of plasmid pPT 0224, in which the (db)₃ unit was assembled prior to incorporation into the genes for DCP-4 and DCP-5.

Figure 4 illustrates the construction of the d₄ subunit which was subsequently incorporated into the DCP-6 monomer unit.

Figure 5 illustrates the construction of the DCP-4, DCP-5, and DCP-6 monomer units by the insertion of multimerized (db)₃ units (DCP-4 and DCP-5) or d₄ units (DCP-6) into the pPT 0140 vector.

Figure 6 illustrates the construction of plasmid vectors for the expression in E.coli of genes for DCP-1, DCP-2, DCP-3, DCP-4, DCP-5 and DCP-6. For this purpose, the respective cloned monomer units were self-ligated to form multimers, then the multimers were inserted into the expression vector pSY 1262.

### DETAILED DESCRIPTION OF THE INVENTION

The collagen-like polymers of the current invention are compatible with self-assembly into triple helix structures either with themselves or other triple helix-forming polymers. For example, native collagen may be used as an "extender" in blends with the collagen-like polymers of the current invention. The compositions of the current invention were specifically designed to be processable into shaped articles, such as fibers and to overcome the problems in processing native collagen, discussed above. The collagen-like polymers of the current invention may also be useful as coating compositions.

Specifically, the polymers of the current invention are block copolymers in which the blocks are either strongly triple helix-forming or generally triple helix-compatible or a mixture of the two, interspersed in a regular way with triple helix-compatible blocks which incorporate structural features designed to promote processability.

All blocks in the collagen-like polymers of the current invention have amino acid sequences comprising multiple repeats of the tripeptide

Gly-x-y,

where
x = a single amino acid, and
y = a single amino acid.

In strongly triple helix-forming blocks, a very high percentage (over 50%) of the amino acids x and y are Pro (or Hyp), and the x and y that are not Pro (or Hyp) are amino acids like Gly, Ala, and Ser which have small, non-bulky side chains and offer no steric hindrance to triple helix formation. The high concentration of proline (or hydroxyproline) and the absence of bulky side chains make the triple helix geometry so favorable thermodynamically for strongly triple helix-forming blocks that even short segments will spontaneously form the characteristic triple helix in solution.

In triple helix-compatible blocks, the amino acids x and y are still likely to be Pro, Hyp, Gly, Ala, or Ser. This makes the triple helix geometry thermodynamically favorable for triple helix-compatible blocks, especially if neighboring blocks are strongly triple helix-forming. Additionally, however, triple helix-compatible blocks can include a limited number of amino acids x and y which are not Pro, Gly, Ala, or Ser, in order to impart useful chemical, physical, or biological properties to polymers which contain them, or to produce amino acid sequences whose corresponding DNA sequences are not excessively repetitious.

In triple helix-compatible blocks which incorporate structural features designed to promote processability, the amino acid compositions are similar to those for the above-described plain triple helix-compatible blocks, but the amino acids x and y which are not Pro, Gly, Ala, or Ser are chosen to provide pH-dependent control of solubility through the presence of ionizable groups, or to provide reactive sites for derivatization for control of solubility. The amino acids may also be chosen to provide reactive sites for covalent or ionic chemical cross-linking in order to provide control of solubility and control and stabilization of chain conformation and aggregation.

More specifically, the collagen-like polymers of the current invention are block copolymers comprised of three types of blocks, A, B, and C, arranged according to the following general formula:

[ C_{f1} ( Aⱼ₁ Bⱼ₂ )ₖ C_{f2} ]ₘ

where f1, k, f2 and m are each equal to or greater than one; and the sum of j1 and j2 is equal to or greater than one. It is understood that f1, k, f2, m, j1 and j2 represent integers.

The collagen-like polymer has an overall size of at least 250 amino acids and preferably a size of about 1000-1100 amino acids, like natural collagen. Preferably, too, j1, j2, and k are chosen so that there are 200-250 amino acids in A or B-type blocks between occurrences of C-type blocks. This provides the kind of long-range order or periodicity which seems to exist in natural collagen, as evidenced by the staggered alignment of tropocollagen molecules in natural collagen fibrils.

All of the A-type blocks in the above general formula (i.e., when j1 > 1) may be identical, but they may also vary among themselves, provided they are all A-type. Similarly, B-type and C-type blocks in same-type runs may be identical or may vary among themselves. The constructs of this invention offer advantages for gene stability and protein expression by avoiding monotonous repetition of individual peptide sequences.

All three types of blocks, A, B, and C, have the general formula:

[Gly - x - y]₃₋₃₀.

Due to the gene stability problems which are known to be posed by long runs of monotonously-repeated small block units, the collagen-like polymers of the current invention are constructed from units large enough to provide structural variety and to allow for "natural" incorporation of groups for cross-linking, bioactivity, and control of high-level structure. Although long synthetic DNA oligomers can be constructed, lengths corresponding to 3 - 100 or less amino acids can be readily constructed using known methods. Block units larger than about a dozen amino acids complicate the construction of the corresponding DNA oligomers. Block polypeptide units of nine to thirty amino acids are preferred.

A-type blocks are strongly triple helix-forming. About 50 to 75% of the amino acids, x and y, are independently selected from the group consisting of Pro and Hyp. Preferably, all of the amino acids, x and y, which are not Pro or Hyp are independently selected from the group consisting of Gly, Ala, and Ser.

An example of a nonapeptide A-type block which is further described in the Examples below is

a = Gly-Ala-Pro-Gly-Pro-Ala-Gly-Pro-Pro.

B-type blocks are triple helix-compatible. At least about 25 to 60% of the x and y are Pro or Hyp, and preferably at least about 66% of the x and y are Gly, Ala, Ser, Pro, or Hyp. The remaining x and y may be any amino acids. Examples of nonapeptide B-type blocks which are further described in the Examples below are b = Gly-Ser-Arg-Gly-Asp-Pro-Gly-Pro-Pro and d = Gly-Ala-Gln-Gly-Pro-Ala-Gly-Pro-Gly. Nonapeptide b was designed to include the bioactive cell binding sequence, Arg-Gly-Asp, while still following the pattern for a triple helix-compatible block. Nonapeptide d was designed to be a triple helix-compatible alternative to the strongly triple helix-forming nonapeptide a, with lower proline content and more variability in the corresponding DNA (achieved by substituting a "wild card" amino acid, Gln, for one of the prolines.)

Other examples of B-type nonapeptides and dodecapeptides which include bioactive cell binding sequences like those found in fibronectin (Arg-Gly-Asp, Arg-Gly-Asp-Ser, Gly-Arg-Gly-Asp, or Gly-Arg-Gly-Asp-Ser) or in laminin (Tyr-Ile-Gly-Ser-Arg) include: 
Gly-Ser-Arg-Gly-Asp-Pro-Gly-Pro-Ala

Gly-Ser-Arg-Gly-Asp-Pro-Gly-Ala-Pro

Gly-Ser-Arg-Gly-Asp-Ala-Gly-Pro-Pro

Gly-Ser-Arg-Gly-Asp-Gly-Gly-Pro-Pro

Gly-Pro-Pro-Gly-Ser-Arg-Gly-Asp-Pro

Gly-Pro-Pro-Gly-Ser-Arg-Gly-Asp-Ala

Gly-Pro-Pro-Gly-Ser-Arg-Gly-Asp-Gly

Gly-Ala-Pro-Gly-Ser-Arg-Gly-Asp-Pro

Gly-Pro-Ala-Gly-Ser-Arg-Gly-Asp-Pro

Gly-Pro-Arg-Gly-Asp-Ser-Gly-Pro-Pro

Gly-Pro-Arg-Gly-Asp-Ser-Gly-Ala-Pro

Gly-Pro-Arg-Gly-Asp-Ser-Gly-Pro-Ala

Gly-Pro-Pro-Gly-Pro-Arg-Gly-Asp-Ser

Gly-Pro-Ala-Gly-Pro-Arg-Gly-Asp-Ser

Gly-Ala-Pro-Gly-Pro-Arg-Gly-Asp-Ser

Gly-Gly-Arg-Gly-Asp-Pro-Gly-Pro-Pro

Gly-Gly-Arg-Gly-Asp-Pro-Gly-Ala-Pro
 
Gly-Gly-Arg-Gly-Asp-Pro-Gly-Pro-Ala

Gly-Gly-Arg-Gly-Asp-Gly-Gly-Pro-Pro

Gly-Gly-Arg-Gly-Asp-Ala-Gly-Pro-Pro

Gly-Pro-Pro-Gly-Gly-Arg-Gly-Asp-Pro

Gly-Ala-Pro-Gly-Gly-Arg-Gly-Asp-Pro

Gly-Pro-Ala-Gly-Gly-Arg-Gly-Asp-Pro

Gly-Pro-Pro-Gly-Gly-Arg-Gly-Asp-Gly

Gly-Pro-Pro-Gly-Gly-Arg-Gly-Asp-Ala

Gly-Gly-Arg-Gly-Asp-Ser-Gly-Pro-Pro

Gly-Pro-Pro-Gly-Gly-Arg-Gly-Asp-Ser

Gly-Tyr-Ile-Gly-Ser-Arg-Gly-Pro-Pro

Gly-Pro-Pro-Gly-Tyr-Ile-Gly-Ser-Arg

Gly-Pro-Pro-Gly-Tyr-Ile-Gly-Ser-Arg-Gly-Pro-Pro

Gly-Pro-Ala-Gly-Tyr-Ile-Gly-Ser-Arg-Gly-Pro-Pro

Gly-Ala-Pro-Gly-Tyr-Ile-Gly-Ser-Arg-Gly-Pro-Pro

Gly-Pro-Pro-Gly-Tyr-Ile-Gly-Ser-Arg-Gly-Ala-Pro

Gly-Pro-Pro-Gly-Tyr-Ile-Gly-Ser-Arg-Gly-Pro-Ala
 
Gly-Ala-Pro-Gly-Tyr-Ile-Gly-Ser-Arg-Gly-Ala-Pro

Gly-Ala-Pro-Gly-Tyr-Ile-Gly-Ser-Arg-Gly-Pro-Ala

Gly-Pro-Ala-Gly-Tyr-Ile-Gly-Ser-Arg-Gly-Ala-Pro

Gly-Pro-Ala-Gly-Tyr-Ile-Gly-Ser-Arg-Gly-Pro-Ala

Gly-Pro-Pro-Gly-Pro-Pro-Gly-Tyr-Ile-Gly-Ser-Arg

Gly-Pro-Ala-Gly-Pro-Pro-Gly-Tyr-Ile-Gly-Ser-Arg

Gly-Ala-Pro-Gly-Pro-Pro-Gly-Tyr-Ile-Gly-Ser-Arg

Gly-Pro-Pro-Gly-Ala-Pro-Gly-Tyr-Ile-Gly-Ser-Arg

Gly-Pro-Pro-Gly-Pro-Ala-Gly-Tyr-Ile-Gly-Ser-Arg

Gly-Pro-Ala-Gly-Pro-Ala-Gly-Tyr-Ile-Gly-Ser-Arg

Gly-Ala-Pro-Gly-Pro-Ala-Gly-Tyr-Ile-Gly-Ser-Arg

Gly-Pro-Ala-Gly-Ala-Pro-Gly-Tyr-Ile-Gly-Ser-Arg

Gly-Ala-Pro-Gly-Ala-Pro-Gly-Tyr-Ile-Gly-Ser-Arg

Gly-Tyr-Ile-Gly-Ser-Arg-Gly-Pro-Pro-Gly-Pro-Pro

Gly-Tyr-Ile-Gly-Ser-Arg-Gly-Ala-Pro-Gly-Pro-Pro

Gly-Tyr-Ile-Gly-Ser-Arg-Gly-Pro-Ala-Gly-Pro-Pro

Gly-Tyr-Ile-Gly-Ser-Arg-Gly-Pro-Pro-Gly-Ala-Pro
 
Gly-Tyr-Ile-Gly-Ser-Arg-Gly-Pro-Pro-Gly-Pro-Ala

Gly-Tyr-Ile-Gly-Ser-Arg-Gly-Pro-Ala-Gly-Pro-Ala

Gly-Tyr-Ile-Gly-Ser-Arg-Gly-Ala-Pro-Gly-Pro-Ala

Gly-Tyr-Ile-Gly-Ser-Arg-Gly-Pro-Ala-Gly-Ala-Pro,
 and

Gly-Tyr-Ile-Gly-Ser-Arg-Gly-Ala-Pro-Gly-Ala-Pro

C-type blocks are triple helix-compatible with functionality for improved processability. About 25 to 60% of the amino acids, x and y, are independently selected from the group consisting of proline and hydroxyproline. At least one of the remaining amino acids, x and y, is independently selected from the group consisting of aspartic acid, glutamic acid, lysine, arginine, histidine, tyrosine, and cysteine to provide pH-dependent ionic charge for control of solubility and/or from the group of lysine, cysteine, tyrosine, aspartic acid, and glutamic acid to provide reactive sites for derivatization or cross-linking. An example of a nonapeptide C-type block which is further described in the examples below is c = Gly-Ala-His-Gly-Pro-Ala-Gly-Pro-Lys. Nonapeptide c is triple helix-compatible and includes an amino acid with a side chain that ionizes as pH drops below neutral (histidine, pKa = 6.0) for pH-dependent control of solubility, and an amino acid with crosslinkable functionality in its side chain (lysine).

Other examples of C-type nonapeptide blocks are shown in the following series: 
Gly-Pro-Lys-Gly-Lys-Ala-Gly-Pro-Lys

Gly-Pro-His-Gly-Lys-Ala-Gly-Pro-Lys

Gly-Pro-His-Gly-Lys-Ala-Gly-Pro-His

Gly-Pro-His-Gly-His-Ala-Gly-Pro-His

Gly-Pro-Glu-Gly-His-Ala-Gly-Pro-Lys

Gly-Pro-Glu-Gly-His-Ala-Gly-Pro-His

Gly-Pro-Glu-Gly-His-Ala-Gly-Pro-Glu,
 and Gly-Pro-Glu-Gly-Glu-Ala-Gly-Pro-Glu The peptides in this series form a progression from a block which has a high density of positive ionic charge except in very basic (high-pH) media to a block which has a high density of negative ionic charge except in fairly acidic (low-pH) media. The series illustrates how amino acids can be chosen in C-type blocks to provide a specific distribution of ionic charge over a specific range of pH for control of polymer solubility.

From these nonapeptide block units, designs for six collagen-like polymers, each comprising about 1000 amino acids, were developed having the structures shown below:

DCP-1 = [c₂ a₂₄ c₂]₄

DCP-2 = [c₂ a₁₂ c₂]₈

DCP-3 = [c₂ ( a b )₁₂ c₂]₄

DCP-4 = [c₂ ( d b )₁₂ c₂]₄

DCP-5 = [c₂ ( d b )₆ c₂]₈,

and

DCP-6 = [c₂ d₂₄ c₂]₄

DCP-1 was designed to be a generic collagen analog, with long runs of triple helix interrupted by infrequent cross-linking and solubility control sites.

The second construct, DCP-2, was designed as a variation on DCP-1, with shorter (half-size) runs of triple helix and twice as many cross-linking/solubility control sites.

The third construct, DCP-3, was designed to include the bioactive cell binding site, Arg-Gly-Asp, from subunit b.

The fourth construct, DCP-4, was also designed to include the bioactive cell binding site, Arg-Gly-Asp, from subunit b. As compared with DCP-3, however, DCP-4 was designed to have a lower proline content and more diversity in the corresponding DNA sequence through the inclusion of subunit d, a B-type block, instead of subunit a, an A-type block.

The fifth construct, DCP-5, was designed as a variation on DCP-4, with shorter (half-size) runs of triple helix-compatible blocks and twice as many cross-linking/solubility control sites. DCP-5 is to DCP-4 as DCP-2 is to DCP-1.

The sixth construct, DCP-6, was designed as a variation on DCP-1 using the triple helix-compatible subunit d in place of the strongly triple helix-forming subunit a.

It is recognized that the above-described constructs each contain about 1000 amino acids and are preferred, but the total number of amino acids may be greater or less depending upon the desired molecular weight (MW) of the construct. Other constructs include, for example:

DCP-1 = [c₂ a₂₄ c₂]₁₋₈

DCP-2 = [c₂ a₁₂ c₂]₂₋₁₆

DCP-3 = [c₂ (ab)₁₂ c₂]₁₋₈

DCP-4 = [c₂ (d b)₁₂ c₂]₁₋₈

DCP-5 = [c₂ (d b)₆ c₂]₂₋₁₆,

and

DCP-6 = [c₂ d₂₄ c₂]₁₋₈

In some cases, it may not be possible to prepare all members of a series using a single cloning strategy. For example, the DNA template for DCP-1 was unstable in cloning and it was possible to isolate only [c₂ a₂₄ c₂]ₙ where n=1.0 - 1.5. In constructing the gene for DCP-2, [c₂ a₁₂ c₂]ₙ was found to be stable in cloning and expression up to n=7, but [c₂ a₁₂ c₂]₈, was apparently unstable and could not be isolated. Similarly, full-length expressions may not be possible in a single expression system. For example, it may not be possible to obtain DCP-2 above n = 5 using the cloning strategy in Example 2. The gene for DCP-3 was readily constructed and was stable in expression, although expression levels were low (1-2%), and the product included lower molecular weight polymer fragments. Similarly, the genes for DCP-4, DCP-5, and DCP-6 were readily constructed and were stable in cloning and expression, and expression levels are discussed in Example 3.

It is recognized that constructs for other collagen-like polymers using A, B and C-type blocks, may be designed. For example, collagen-like polypeptides characterized by the formula, [Aⱼ₁ Bⱼ₂]ₖ, where k is equal to or greater than one, and the sum of j1 and j2 is equal to or greater than one, may be prepared. Collagen-like polypeptides containing only C-type blocks may also be prepared. Another design for a collagen-like block copolymer is represented by the formula, [C_{f1}(Aⱼ₁Bⱼ₂)ₖC_{f2}]ₘ, where the sum of f1 and f2 is equal to or greater than one, the sum of j₁ and j₂ is equal to or greater than one, and k and m are each equal to or greater than one.

### METHODS FOR THE PRODUCTION OF HIGH MOLECULAR WEIGHT COLLAGEN-LIKE PROTEIN POLYMERS

### EXAMPLE 1

### DNA Preparation Methods

1. Preparation of plasmid DNA from E. coli.
   A. Small scale. Plasmid DNA was prepared from 1.5 ml cultures by either the boiling procedure or the alkaline lysis method (Maniatis et al., Molecular Cloning: A Laboratory Manual. Cold Spring Harbor Laboratory, Cold Spring Harbor. (1982).
   B. Large scale. A plasmid-carrying strain was grown overnight in 1 liter of Luria broth with the appropriate antibiotic. The cells were collected by centrifugation at 10,000xg for 5 min and resuspended in 10 ml of ice cold TE (10 mM Tris-HCl pH 8, 1 mM EDTA). The cells were centrifuged again, resuspended in 4 ml of TES (TE and 25% w/v sucrose) and homogenized by vortexing. The samples were kept on ice for the following steps. Lysozyme (1 ml of 10 mg/ml) was added to the cell suspension and incubated for 5 min before the addition of 2 ml of 0.5 M EDTA pH 8. After 10 min incubation, 50 ml of proteinase K (40 mg/ml) were added followed 10 min later with 15 ml of lysing buffer (0.1% Triton X-100, 1 mM EDTA, 50 mM Tris-HCl pH 8). After 15-20 min, the cell lysate was centrifuged at 35,000xg for 90-120 min. The supernatant (19.8 ml) was transferred to a plastic tube with 20 mg of CsCl and 400 ul of ethidium bromide (10 mg/ml). After dissolution, the mixture was divided into two polyallomer ultracentrifuge tubes, sealed with heat and centrifuged in a Beckman Ti 65 rotor at 60,000 rpm for 24 hr. The lower plasmid DNA band was removed from the tube with a hypodermic needle. The ethidium bromide was extracted three times with an equal volume of NaCl-saturated isopropanol. Two volumes of H₂O were added to the DNA solution, and then the DNA was precipitated with ethanol.

2. Deproteinization.
   Phenol extraction was performed on a convenient volume of DNA sample, typically between 100 ul to 10 ml. The DNA sample was diluted in 0.01 M TRIS-HCl pH 7.5,1 mM EDTA and an equal volume of water-saturated phenol was added. The sample was vortexed briefly and placed on ice for 3 min. After centrifugation for 3 min in a microfuge, the aqueous layer was removed to a new tube and extracted once with an equal volume of chloroform:isoamylalcohol (24:1).
3. Ethanol precipitation.
   DNA in an aqueous buffer was concentrated by ethanol precipitation. To the DNA sample was added 1/10 volume of 3 M sodium acetate pH 7.5 and 2-3 volumes of cold ethanol. The DNA was precipitated for 30 min at -70 °C or overnight at -20 °C and then pelleted by centrifugation in the microfuge for 15 min at 4 °C. The pellet was washed once with 200 ul of cold 80% ethanol and precipitated again for 10 min at 4°C. After air drying or lyophilization, the pellets were resuspended in the appropriate buffer.
4. Phosphatase treatment of DNA. Phosphatase treatment of DNA was performed by adding 1 ul (25 units) of calf intestinal phosphatase (Boehringer Mannheim) directly to the restriction enzyme digestion reaction and continuing the incubation for 30 min at 37°C. The phosphatase was inactivated for 60 min at 65°C prior to deproteinization by phenol extraction.
5. Fill-in reaction with DNA polymerase 1.
   DNA was resuspended in buffer containing 50 mM Tris-HCl pH 7.4, 50 mM KCl, 5 mM MgCl₂, and 400 mM each of the four deoxynucleotide triphosphates. Ten units of Klenow DNA polymerase (BRL) were added, and the reaction was allowed to proceed for 15 min at room temperature. The DNA was then phenol extracted and ethanol precipitated.
6. Digestion with restriction endonucleases.
   DNA was digested with restriction endonucleases (REN) in 1 x "AA" buffer [10 x AA buffer is 330 mM Tris-acetate, pH 7.9, 660 mM potassium acetate, 100 mM magnesium acetate, 50 mM dithiothreitol (DTT) and 1 mg/ml bovine serum albumin (nuclease free)]. Whenever possible, the concentration of DNA was kept below 1 ug/25 ul. Incubation was at 37°C for 1-4 hrs for most restriction endonucleases except for Ball, BanI and NaeI digestions which were incubated overnight.
7. Analytical agarose gel electrophoresis of DNA. To DNA samples for gel analysis was added 0.2 volumes of loading buffer (5 x electrophoresis buffer, 0.01% bromphenol blue dye, 50 mM EDTA, and 50% glycerol). Then the samples were loaded into lanes of a horizontal submerged electrophoresis unit containing a 1.0% (w/v) agarose gel. The electrophoresis buffer was either 1 x TAC or 1/2 x TBE. The 1 x TAC is 40 mM Tris-base, 10 mM EDTA, adjusted to pH 7.8 with acetic acid. The 1/2 x TBE is 0.045 M Tris-base, 0.045 M boric acid, 1 mM EDTA, pH 8. The gel was run at 40-50 V for 18 hr, then removed and stained with 0.5 ug/ml ethidium bromide for 30 min. The DNA bands were visualized on a long wavelength UV transilluminator.
8. Preparative agarose gel electrophoresis.
   The procedures and materials are the same as for the analytical agarose gel electrophoresis. The only difference is the use of low melting point (LMP) agarose, ranging in concentration from 0.5 to 2.5% (w/v) depending on the size of the DNA fragment to be purified. DNA restriction fragments were excised from the LMP agarose gels after visualization with ethidium bromide. For agarose ligation the buffer used was 1X TAE (50 mM Tris Acetate)
9. NACS purification.
   Gel fragments containing DNA were melted at 70°C for 5 min and diluted approximately 5 fold with TE1 (10 mM Tris-HCl pH 7.5, 0.2 M NaCl). The gel solution was applied to a NACS column (BRL). The column was washed with 5 ml of the same buffer. The bound DNA was eluted with 300 ul of either TE2 (10 mM Tris-HCl pH 7.5,1.0 M NaCl) for DNA fragments smaller than 1000 bp or TE3 (10 mM Tris-HCl pH 7.5, 2M NaCl) for larger fragments. The eluted DNA was concentrated by ethanol precipitation.
10. DNA ligation.
   Reactions for ligating cohesive ends contained 1 ug DNA, 1 x AA buffer (see step 6, above) 1 mM ATP and 20 units of T4 DNA ligase (BRL) in a 20 ul final reaction volume. The ligation was allowed to proceed for 16-18 hr at 15°C or 1-2 hr at room temperature. For blunt-ended ligations the reactions contained 1 ug DNA, 25 mM Tris-HCl pH 7.5, 5 mM MgC12, 5 mM DTT, 0.25 mM spermidine, 200 ng BSA, 1 mM hexamine cobalt chloride (HCC), 0.5 mM ATP and 400 units T4 DNA ligase (NEB) in a 20 ul reaction volume. The ligation was allowed to proceed for 30 min to 1 hr at room temperature.
11. Agarose DNA ligation.
   The agarose was melted at 65° C, the temperature was then lowered to 37° C and ligation buffer ( 5X = 100 mM Tris-HCl, pH 7.5, 50 mM MgCl2, 50 mM DTT, 1 mM ATP) was added; the tube was then placed at room temperature and Ligase was added (1000 units T4 DNA ligase (NEB)), the reaction volume was usually 50 ul. The reaction was incubated at 15° C for 16-18 hrs.

### mRNA Methods

1. Preparation of mRNA. mRNA was prepared using a modified procedure described by Summers, W.C. (Anal. Biochem. 33: 459-463 (1970)). Cells were grown at 30°C or 42°C in LB medium supplemented with Kanamycin (50 ug/ml). 10 ml of cells at OD₆₀₀ 1.0 were spun and the cell pellet was resuspended in 10 ml of protoplasting buffer ( 15 mM Tris-HCl pH 8.0, 0.45 M sucrose, 8 mM EDTA); 80 ul of Lysozyme at 50 mg/ml were added and the mixture was then incubated on ice for 15 min. The cell suspension was spun for 5 min. at 7,000 RPM in an SS-34 rotor using an RC-5B centrifuge. The pellet was resuspended in 0.5 ml of lysing buffer (10 mM Tris-HCl pH 8.0,10 mM NaCl, 1 mM Na-Citrate, 1.5 % w/v SDS) and 15 ul of diethylpyrocarbonate (DEPC). The suspension was mixed gently and then transferred to a 1.5 ml Eppendorf tube, incubated at 37°C for 5 min. and then chilled on ice. 250 ul of saturated NaCl ( 40% w/v) were added, mixed gently, and the incubation, on ice, was prolonged for an additional 10 min. The slurry was spun at 4°C for 15 min. The supernatant was placed in two 1.5 ml tubes and 1 ml of 100% ethanol was added to each. The RNA was precipitated in the cold and then spun at 4°C for 20 min. The pellets were rinsed in 70% ethanol and dried. The RNA was then resuspended in 0.1 ml H₂O and OD₂₆₀ and OD₂₈₀ were taken to measure the recovery and purity of the RNA. The average recovery of total RNA ( 5% mRNA, 95% tRNA + rRNA), from 10 ml of growing cells,was between 0.5 and 1.0 mg.
2. Northern blot analysis.
   The RNA, purified as described above, was run on agarose gel and transferred to nitrocellulose following the procedure described in Maniatis et al., Molecular Cloning: A Laboratory Manual. Cold Spring Harbor Laboratory, Cold Spring Harbor (1982). pp. 202-203, using 10X SSC as transfer buffer. The ECL gene detection system kit (Amersham) was used for labeling of the probe, hybridization conditions and detection. The procedures were executed as described in: ECL gene detection system RPN2101, version 2, Amersham International plc.

### Bacterial Transformation Methods

1. Preparation of transformation-competent E. coli cells. A culture of 200 ml of sterile L broth was inoculated with a small loopful of E.coli cells. This was incubated with shaking at 37°C until the OD₆₀₀ was approximately 0.5. The culture was placed on ice for 10 min and centrifuged at 6,000xg for 10 min. The cell pellet was resuspended in 100 ml of ice-cold 0.1 M MgCl₂, kept on ice for 30-40 min and centrifuged again. The pellet was resuspended in 2 ml of ice-cold 0.1 M CaCl₂, transferred to a sterile test tube and incubated on ice for 24 hr. The competent cells were then aliquoted and stored at -70°C.
2. Transformation of E. coli.
   An aliquot of frozen competent cells was thawed on ice. To 50 ul of cells, 0.1 to 1 ug of DNA was added and the mixture was incubated on ice for 30 min. The tube was removed from ice and placed in a 42°C bath for 2 min. L broth (1 ml) was added and the transformation mix incubated with shaking at the desired temperature (usually 30°C or 37°C) for 2 hr. Then one-tenth of the transformation was plated on L broth plates containing the appropriate antibiotic and, when necessary, XGAL and IPTG were added. Antibody Production, Protein Chemistry and Electrophoresis of Proteins

1 . Preparation of Antibody to Artificially Synthesized Peptides.
   Synthetic peptide of sequence Gly-Ala-His-Gly-Pro-Ala-Gly-Pro-Lys-Gly-Ala-His-Gly-Pro-Ala -Gly-Pro-Lys-Gly-Ala-Pro-Gly-Pro-Ala-Gly-Pro-Pro-Gly-Ala-Pro-Gly-Pro-Ala-Gly-Pro-Pro (DCP-c₂a₂) was coupled to keyhole limpet hemocyanin for use as an immunogen. The material was sent to Antibodies, Inc. for preparation of antibodies in rabbits. Peptide conjugates at a concentration of 1 mg/ml in complete Freund's adjuvant were used to immunize rabbits at day 0. Animals were re-injected with antigen in Freund's incomplete adjuvant at day 30 and titered at day 60. Positive sera was detected using a microtiter-RIA using the synthetic peptide as antigen. Kagen and Glick (1979), in Methods of Radioimmunoassay, Jaffe and Berman (eds.), Academic Press, p 328. Antisera was obtained that reacted with synthetic peptides of the DCP-1 and DCP-2 sequences.
   Following the procedure described above one additional peptide was synthesized having the formula (Gly-Ala-Pro-Gly-Pro-Ala-Gly-Pro-Pro-Gly-Ser-Arg-Gly-Asp-Pro-Gly-Pro-Pro)₂ (DCP-(ab)₂ ), which was also coupled to keyhole limpet hemocyanin for use as an immunogen.
   Polyclonal antisera were then prepared as described above which bound to the synthetic peptide of the DCP-3 sequence.
2. Polyacrylamide gel electrophoresis of proteins.
   Approximately 10⁹ E. coli cells from growing cultures were pelleted by centrifugation at 10,000xg for 5 min. The cell pellets were resuspended in 100 to 500 ul of 2X sample buffer (100 mM Tris-HCl pH 6.8, 4% SDS, 10% B-mercaptoethanol, 60% glycerol or sucrose) and sonicated for 30 sec using a Tekmar sonic disruptor. Samples were boiled for approximately 5 min and 20 to 100 ul of the cell lysates were loaded on an SDS-polyacrylamide gel (7.5 to 16% w/v). The gels were prepared following the procedure of Laemmli (Nature, 27: 80-685 (1970)). The proteins in the gels were stained with 2% Coomassie brilliant blue in 10% methanol, 7.5% acetic acid for 1 hr and destained in 10% methanol, 7.5% acetic acid overnight.
3. Protein expression analysis.
   An overnight culture which had been grown at 30° C was used to inoculate 50 ml of media contained in a 250 ml flask. Kanamycin was added at a final concentration of 50 ug per ml and the culture was incubated with agitation (200 rpm) at 30° C. When the culture reached an OD₆₀₀ of 0.8, 40 ml were transferred to a new flask prewarmed at 42° C and incubated at the same temperature for approximately 2 hours. The cultures (30° and 42°) were chilled on ice and OD₆₀₀ was taken. Cells were collected by centrifugation divided in 1.0 OD₆₀₀ aliquots and used to perform western analysis using the appropriate antibodies.
4. Immunoblotting of proteins in gels.
   After protein electrophoresis, one of the flanking glass plates was removed from the polyacrylamide gel. The gel surface was wetted with transfer buffer (25 mM Tris-HCl, 192 mM glycine, 20% methanol). A piece of nitrocellulose paper ( Sartorius, SM11307 ) was saturated with transfer buffer and laid on the gel. Air bubbles between the filter and the gel were removed. The gel and nitrocellulose filter were placed in the transfer unit as specified by manufacturer (Bio-Rad). Transfer was allowed to proceed at 200 mA for 3-4 hr. Then the nitrocellulose filter was removed and stained with Amido-Schwartz for 3 min ( 0.05% Amido black, 45% deionized H₂O, 45% methanol, 10% acetic acid) and destained in H₂O. The filter was incubated for at least 10 min at room temperature in "BLOTTO" (5% w/v nonfat dry milk, 50 mM Tris-HCl pH 7.4, 0.9% w/v NaCl, 0.2% w/v sodium azide). The filter was placed in serum appropriately diluted ( 1: 50 to 1: 500 ) in 0.5X Blotto (2.5% nonfat dry milk, 50 mM Tris-HCl pH 7.4, 0.9% NaCl, 0.2% sodium azide) and was gently agitated for approximately 16 hr at room temperature. The filter was washed for 1 hr with 5 changes of TSA ( 50 mM Tris-HCl pH 7.4, 0.9% NaCl, 0.2% sodium azide). The blot was placed in 15 ml of 0.5X BLOTTO solution containing 1x10⁷ cpm of the ¹²⁵I protein A and gently agitated for 2 hrs at room temperature. The filter was washed for 2 hrs with a minimum of 7 changes of TSA, rinsed once with deionized H₂O and air dried. The blot was covered with Saran wrap and autoradiographed.
5. Amino Acid Analysis.
   Amino acid compositions are determined by the PTC derivatization procedure of Henrickson and Meredith (1984). Protein samples were hydrolysed with 5.7 N constant boiling HCl at 108°C for 24 hours in vacuo. After reaction with PITC, amino acid derivatives were detected at 254 nm by HPLC reverse phase chromatography using a Waters 100E system and a Supelco C18 column (4.6 mm x 25 cm) with a linear gradient of 0-50% acetonitrile in 0.1 M NH₄OAc pH 6.78 as a mobile base. Henrickson, R.L. and Meredith, S.C. (1984) Amino Analysis by Reverse Phase High Performance Liquid Chromatography., Anal. Biochem. 137:65-74.
6. Peptide Synthesis.
   Synthetic peptides were prepared by solid phase synthesis on an Applied Biosystems Model 430A Peptide Synthesizer using the standard symmetric anhydride chemistry as provided by the manufacturer. The coupling yield at each step was determined by the quantitative ninhydrin procedure of Sarin et al., (1981). The synthetic peptide was cleaved from the solid support and amino acid blocking groups were removed using anhydrous HF (Stewart and Young, 1984). Crude peptides were desalted by chromatography over Sephadex G-50 (Sarin, V.K., Kent, S.B.H., Tam, J.P. and Merrifield, R.B. (1981). Anal. Biochem. 237:927-936. Stewart, J.M. and Young, J.D. (1984). Solid Phase Peptide Synthesis, Pierce Chemical Company, Rockford, IL. pp 85-89).

### Synthetic DNA Methods

1. In vitro DNA synthesis.
   The N,N-diisopropylphosphoramidites, controlled-pore glass columns and all synthesis reagents were obtained from Applied Biosystems, Foster City, California. Synthetic oligonucleotides were prepared by the phosphite triester method with an Applied Biosystems Model 381A DNA synthesizer using a 10-fold excess of protected phosphoramidites and 1 umole of nucleotide bound to the synthesis support column. The chemistries used for synthesis are the standard protocols recommended for use with the synthesizer and have been described (Matteucci et al,, J. Amer. Chem. Soc., 103:3185-3319 (1981)). Deprotection and cleavage of the oligomers from the solid support were performed according to standard procedures as described by McBride et al. Tetrahedron Letters, 24:245-248 (1983). The repetitive yield of the synthesis as measured by the optical density of the removed protecting group as recommended by Applied Biosystems (1984) was greater than 97.5%. The crude oligonucleotide mixture was purified by preparative gel electrophoresis as described by the Applied Biosystems protocols of November 9, 1984 (User Bulletin No. 13). The acrylamide gel concentration varied from 10 to 20% depending upon the length of the oligomer. The purified oligomer was identified by UV shadowing, excised from the gel and extracted by the crush and soak procedure (Smith, Methods in Enzymology. 65:371-379 (1980)).
2. Sequencing of DNA. DNA sequences were determined by the following methods. Fragments containing the region of interest were cloned into the multiple cloning site of M13mp18 or M13mp19 (Maniatis et al., 1982, and Norrander et al., 1983). Single-stranded DNA was prepared and sequenced by the primer extension method (Sanger et al., 1977 and Biggin et al., 1983) using ³⁵S-deoxyadenosine 5'-(alpha-thio)-triphosphate (New England Nuclear) as label. In some cases, reverse transcriptase (Molecular Genetics) was used to extend the primer, using the dideoxy:deoxynucleosidetri-phosphate ratios utilized by Zagursky et al., (Gene Anal. Tech. (1985) 2:89-94)-Deoxyadenosine triphosphate labeled with either ³²P or ³⁵S was used in these reactions. Compression artifacts which appeared in some G-C rich sequences were overcome by eliminating deoxyguanosine triphosphate from the G reaction, and using deoxyinosine triphosphate (P-L Biochemicals) at a final concentration of 37.5 uM instead. In the other mixes, the concentration of dideoxyGTP in the G reaction was 0.5 mM. All sequences were run on 6 or 8% polyacrylamide gels containing 8 M urea (Sanger et al 1978). Primers used for sequencing were purchased from P-L Biochemicals. Storage and analysis of data utilized software from DNA Strider, DNA Inspector IIe or DNAid for Apple Macintosh personal computers.
   Dideoxy DNA sequencing of double stranded plasmid DNA. Plasmid DNA was prepared as described previously (Preparation of plasmid DNA from E. coli, Small scale, Maniatis et al.). Primers were synthesized using a DNA synthesizer as described previously, and were annealed to the plasmid DNA following the procedure described above for M13 sequencing. The sequencing reactions were done using Sequenase (United States Biochemicals) and the conditions were as recommended by the supplier. All sequences were run on pclyacrylamide gels as described above.

### EXAMPLE 2

**TABLE 1**

| STRUCTURE OF DCP-1, 2 & 3 | |
|---|---|
| DCP-1 | (c₂a₂₄c₂)₄ |
| DCP-2 | (c₂a₁₂c₂)₈ |
| DCP-3 | (c₂(ab)₁₂c₂)₄ |

where:

a = Gly-Ala-Pro-Gly-Pro-Ala-Gly-Pro-Pro
b = Gly-Ser-Arg-Gly-Asp-Pro-Gly-Pro-Pro
c = Gly-Ala-His-Gly-Pro-Ala-Gly-Pro-Lys

### DNA DESIGN

Due to the complexity of the DCP polymer structures the design of the gene monomers was as follows:
1. design and synthesis of 2 c₂ units (5' and 3')
2. design and synthesis of 2 a units
3. design and synthesis of 2 ab units

### PLASMID pPT 0134 CONSTRUCTION

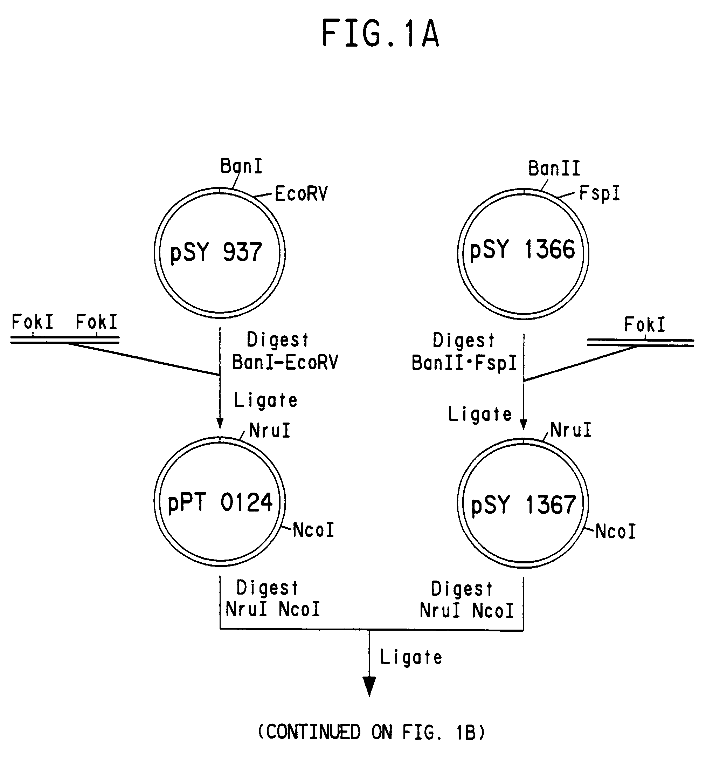

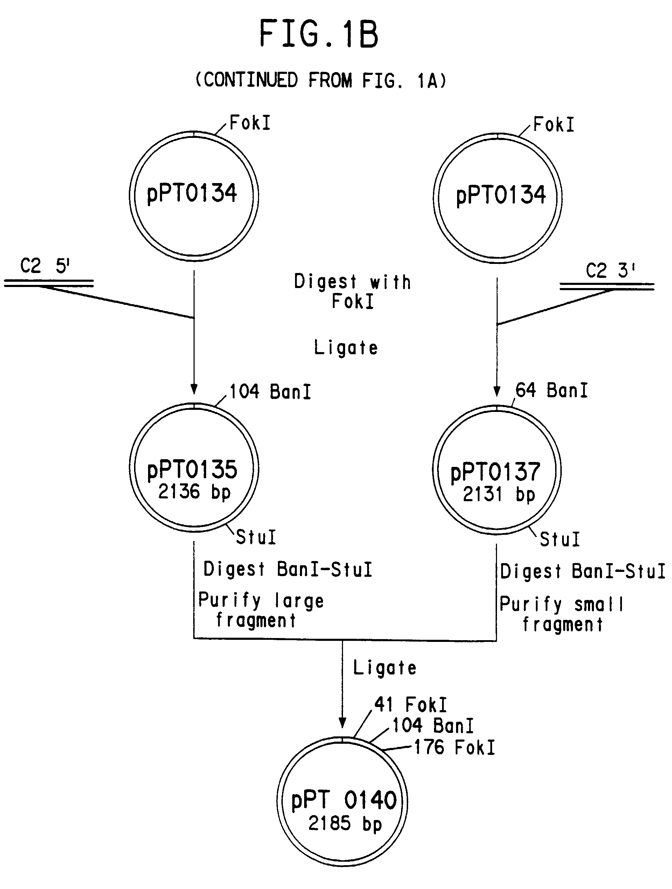

Referring to Figure 1, the acceptor vector PPT 0134 was designed and constructed specifically to accommodate the construction requirements of the DCP polymer genes. This vector contains two recognition sites for FokI REN in its MCS (multiple cloning site). Two oligonucleotide strands containing these sites were synthesized and purified as described in example 1.

FokI FokI ScaI

O.A) 5' - GTGCTGCGGATGCTCGAGATGGTGCATGCATGTACATCCGAGTACTTCGAT
O.B) 3' - ACGCCTACGAGCTCTACCACGTACGTACATGTAGGCTCATGAAGCTA

After annealing, the two oligonucleotide strands were ligated with pSY 937 (see patent application number: PCT/US87/02822) which had been digested with BanI and EcoRY RENs. The product of the ligation mixture was transformed into E. coli and selected on bacterial plates containing the antibiotic chloramphenicol. Plasmid DNA from individual colonies was analyzed on agarose gel electrophoresis after digestion with ScaI and StuI RENs. One plasmid pPT 0124 contained the expected DNA fragment.

The new MCS was then moved to plasmid pSY 1367. This plasmid is a derivative of pSY 1299 (see patent application number: PCT/US87/02822). Plasmid pSY 1299 was digested with NciI REN and the large DNA fragment was purified by agarose gel electrophoresis and NACS purification. The purified DNA fragment was treated with DNA Polymerase (see example 1), ligated, then digested with FokI prior to transformation in E.coli strain HB 101. Plasmid DNA from single colonies was purified and analyzed by restriction digests. One plasmid, pSY 1366, was found to be correct and lacking the only FokI site present in pSY 1299.

Two oligonucleotide strands were synthesized and purified as described in example 1:

(BanII) FokI
1.A) 5' - CTACATGTGTTACACATCCCGTGC
1.B) 3' - CCGAGATGTACACAATGTGTAGGGCACG

Oligonucleotide strands 1.A and 1.B were annealed and ligated with the DNA of plasmid pSY 1366 which had been digested with BanII and FspI RENs. The products of this ligation reaction were transformed into E.coli strain HB101. Plasmid DNA from transformed colonies was purified and digested with FokI. Clones which linearized with FokI were sequenced. Plasmid pSY 1367 contained the desired MCS sequence and was chosen for subsequent constructions

Plasmid pPT 0124 and pSY 1367 were digested with NruI and NcoI and the DNA fragments were purified by agarose gel electrophoresis and NACS purification. The small fragment (approximately 500bp) from pPT 0124 was ligated with the large fragment from pSY 1367. The product of the ligation mixture was transformed into E.coli. Plasmid DNA from single colonies was purified and analyzed by restriction digests and DNA sequencing. One plasmid, pPT 0134, contained the desired sequence and was used as the acceptor vector for the DCP constructions.

### SYNTHESIS AND ASSEMBLY OF THE c UNITS

Four oligonucleotide strands were synthesized and purified as described in example 1. Each pair of oligonucleotide strands encodes a C₂ unit. 
2.A) 5'-GTGCTCACGGCCCAGCAGGTCCGAAGGGCGCGCATGGCCCAGCAGGCCCGAAAGGTGCC
2.B) 3'-    AGTGCCGGGTCGTCCAGGCTTCCCGCGCGTACCGGGTCGTCCGGGCTTTCCACGGCACG

2.C) 5'- GTGCCATGGCCCAGCAGGACCGAAAGGAGCTCACGGTCCGGCAGGTCCGAAAG
2.D) 3' -    GTACCGGGTCGTCCTGGCTTTCCTCGAGTGCCAGGCCGTCCAGGCTTTCCACG
 Oligonucleotide strands 2.A and 2.B were annealed and ligated with the DNA of plasmid pPT 0134 which has been digested with FokI REN. The products of this ligation reaction were transformed into E.coli strain HB101. Plasmid DNA from transformed colonies was purified and digested with SfiI Clones which linearized with SfiI were sequenced. Plasmid pPT0135 contained the desired c₂ sequence and was chosen for subsequent constructions. Strands 2.C and 2.D were annealed, ligated, and transformed as were strands 2.A and 2.B. Plasmid DNA from transformed colonies was purified and digested with BanII REN. Clones which linearized with Ban II were sequenced. Plasmid pPT0137 containing the correct C₂ DNA sequence was used for the assembly of the c₂-c₂ intermediate plasmid construction.

Plasmid pPT 0135 and pPT 0137 were digested with BanI and StuI RENs The large fragment from pPT 0135, containing the C₂ (strands A+B), was ligated with the small fragment of pPT 0137 containing the C₂ fragment (strands C+D). The ligation products were transformed into E.coli strain HB101. Plasmid DNA from transformants was purified and two digestions were performed, SfiI-StuI and BanII-StuI RENs, respectively. Clones that released a DNA fragment of approximately 800 bp in both digestions were sequenced. Plasmid pPT 0140 contained the correct c₂-c₂ sequence, as shown in Table 2, and was used for DCP gene monomer constructions. The construction of pPT 0140 is illustrated in Figure 1.

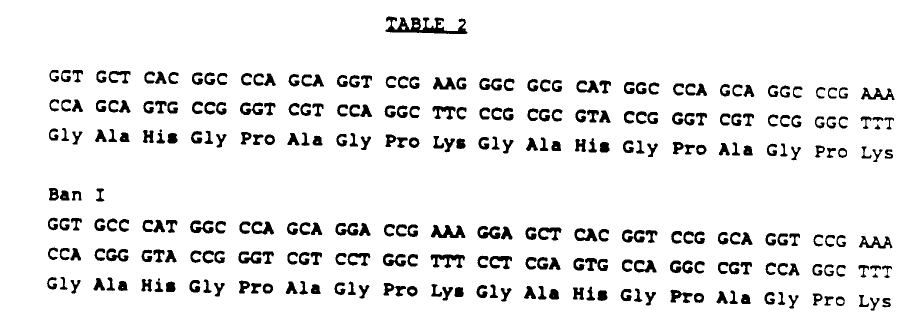

### DCP-1 and 2 GENE MONOMER CONSTRUCTIONS

Two oligonucleotide strands were synthesized and purified as described in example 1.

3.A) 5' - GTGCGCCTGGACCGGCTGGTCCACCGGGTGCTCCGGGACCTGCAGGCCCGCCAG
3.B) 3' -     CGGACCTGGCCGACCAGGTGGCCCACGAGGCCCTGGACGTCCGGGCGGTCCACG

The two oligonucleotide strands encoding a₂ (3A & 3B) were annealed and ligated with plasmid DNA pPT 0134 previously digested with FokI REN. The products of the ligation mixture were transformed into E.coli strain HB101. Plasmid DNA from transformants was digested with PstI REN and clones that were linearized were sequenced. Plasmid pPT 0142 was found to be correct and used for the multimerization of a₂ units.

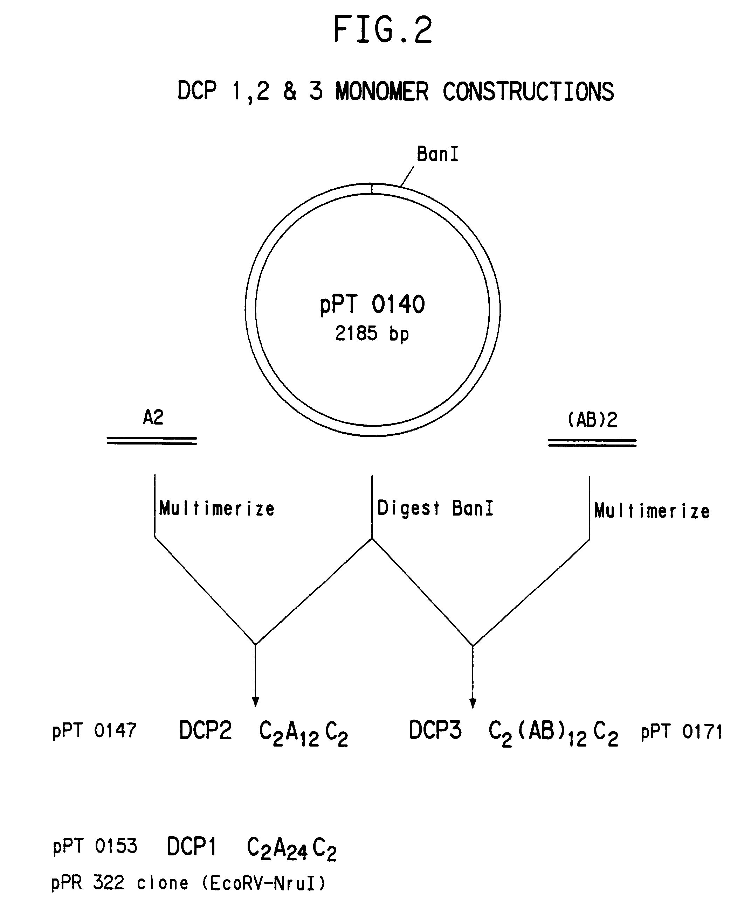

Plasmid DNA pPT 0142 was digested with FokI REN and the fragment containing the a₂ unit was isolated by agarose gel electrophoresis and purified using a NACS column as described (see example 1). The DNA fragment was self-ligated and the products of the ligation were ligated with pPT 0134 previously digested with FokI REN. The products of the ligation mixture were transformed into E.coli strain HB101. Plasmid DNA from transformants was purified and digested with FokI REN. Clones containing DNA inserts of 162 bp corresponding to a₆ were selected for sequence analysis. Plasmid pPT 0144 had the expected DNA sequence and was selected for subsequent constructions. Plasmid DNA from pPT 0144 was digested with FokI REN and the two DNA fragments generated by the digestion were separated using agarose gel electrophoresis. The smaller DNA fragment was further purified using a NACS column ( see example 1 ). As illustrated in Figure 2, the DNA fragment carrying the a₆ coding sequence was ligated with plasmid DNA pPT 0140 previously digested with FokI . The products of the ligation were transformed into E.coli strain HB101. Plasmid DNA from individual colonies was analyzed for inserts containing multiple a₆ DNA fragments by digestion with FokI. Several size inserts were found ranging from a₆ to a₂₄. One clone, pPT 0147 (shown in Table 3) was identified to contain the desired DCP-2 gene monomer sequence c₂a₁₂c₂ and was used for further constructions.

The clone containing the a₂₄ gene monomer (shown in Table 4), necessary to construct the DCP1 polymer gene, was found to be highly unstable during subsequent passages. This instability was attributed to the high copy number of the acceptor plasmid. For this reason, the gene monomer from this plasmid was recloned into pBR 322 (F. Bolivar, et al (1977) Gene 2:95-113)- The plasmid DNA containing the c₂a₂₄c₂ gene fragment was isolated from the plasmid by digestion with NruI and EcoRV RENs, agarose gel electrophoresis, and purified on a NACS column. This DNA fragment was ligated with plasmid pBR322 DNA digested with EcoRV and NruI RENs (see agarose ligation in example 1 ). The products of this ligation were transformed into E.coli strain HB101 and selected on bacterial plates containing the antibiotic ampicillin at 100 ug/ml. Plasmid DNA from individual colonies was analyzed by digestion with BgII REN. Clones containing the insert were further analyzed and one, pPT 0153, was chosen for the construction of the DCP-1 polymer gene. This plasmid was stable.

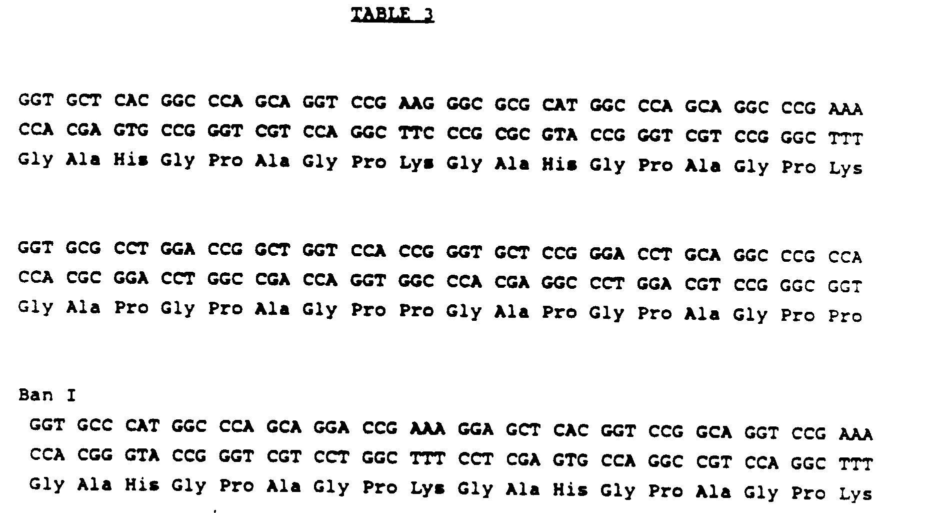

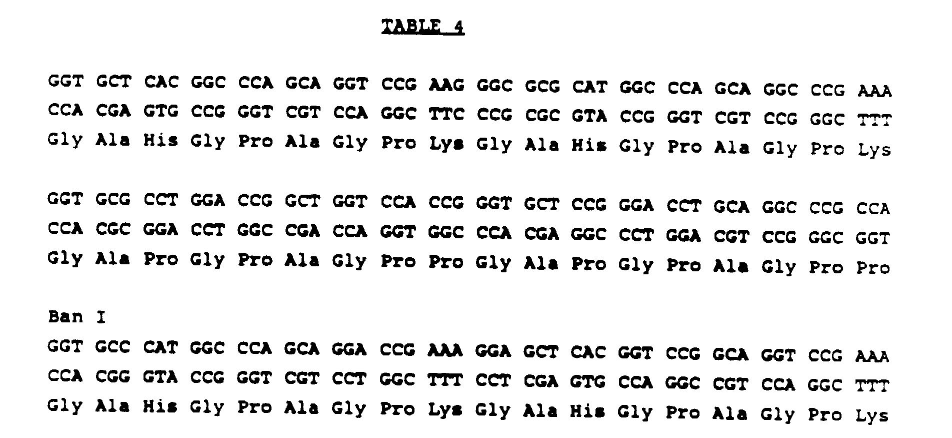

### DCP-1 POLYMER GENE CONSTRUCTION

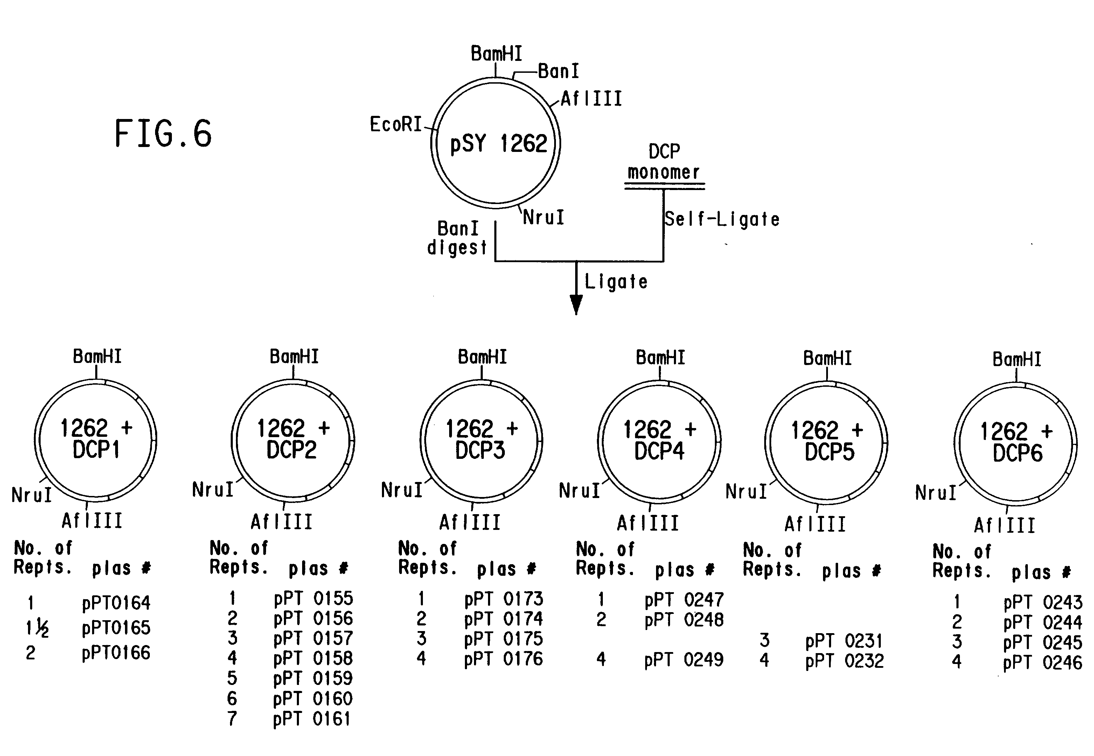

Plasmid DNA pPT 0153 was digested with AcyI REN and subsequently with FokI REN. The DNA fragment containing the DCP-1 gene monomer, 783 bp, was isolated by agarose gel electrophoresis and purified using a NACS column (see example 1 ). The monomer gene fragment was ligated with pSY 1262 (see patent application number: PCT/US87/02822) which had been digested with BanI REN. The ligation product was transformed into E.coli strain HB101 and selected for growth on bacterial plates containing the antibiotic kanamycin. Plasmid DNA from individual colonies was analyzed for inserts containing multiple DCP-1 monomer fragments by digestion with BamHI and PvuII RENs and electrophoresis on agarose gel. One clone, pPT 0164, contained the gene monomer (783 bp); another, pPT 0165, a slightly larger fragment (approximately 1200bp); and a third, pPT 0166, a gene dimer (1566 bp). (See, Figure 6).

### DCP-1 PROTEIN EXPRESSION ANALYSIS

E. coli strain HB101 containing plasmid pPT0164, or pPT 0165 or pPT 0166 was grown at 30° C to an OD₆₀₀ of 0.7 and then shifted to 42° C for 2.0 hours. The proteins produced by these cells were analyzed by western blot analysis for novel protein bands which reacted with DCP peptide specific antisera. A band with an apparent molecular weight of approximately 45 kD was observed in the culture containing the plasmid pPT 0164. A band of 68 kD was observed with pPT 0165 and a light smear of bands with pPT 0166. Because of the low level of detectable expression in the strain containing pPT 0166, the mRNA produced by the DCP-1 clones was analysed. mRNA was prepared as described in example 1. By northern blot analysis (see example 1 ), the DCP specific mRNA from all clones was shown to be full length and synthesized at approximately the same level regardless of the DCP gene size. The probe used for this analysis was the DCP-2 monomer DNA fragment.

DCP-1    pPT 0166       561 Amino Acids       MW:46,409 Dalton

Met-Asp-Pro-Val-Val-Leu-Gln-Arg-Arg-Asp-Trp-Glu-Asn-Pro-Gly-
Val-Thr-Gln-Leu-Asn-Arg-Leu-Ala-Ala-His-Pro-Pro-Phe-Ala-Ser-
Asp-Pro-Met

[(Gly-Ala-His-Gly-Pro-Ala-Gly-Pro-Lys)2
(Gly-Ala-Pro-Gly-Pro-Ala-Gly-Pro-Pro)24
(Gly-Ala-His-Gly-Pro-Ala-Gly-Pro-Lys)2]2

Gly-Ala-Met-Asp-Pro-Gly-Arg-Tyr-Gln-Leu-Ser-Ala-Gly-Arg-Tyr-
His-Tyr-Gln-Leu-Val-Trp-Cys-Gln-Lys

### DCP-2 POLYMER GENE CONSTRUCTION

Plasmid DNA from pPT 0147 was digested with FokI REN and the digestion fragments were separated by agarose gel electrophoresis. The DCP-1 gene fragment of 483 bp was excised and purified by NACS column (See Example 1). As illustrated in Figure 6, the purified fragment was ligated with pSY 1262 which had been digested with BanI REN. The products of this ligation were transformed into E.coli strain HB101 and the transformants were selected for growth on bacterial plates containing the antibiotic kanamycin. Plasmid DNA from individual colonies was purified and analyzed for multiple insertions of the DCP-2 gene monomer fragment. Several clones were obtained ranging in size from 500 to 3,000 bp. See Table 5 for result.

### DCP-2 PROTEIN EXPRESSION ANALYSIS

E. coli strain HB101 containing plasmids pPT 0155 to 0163 were grown at 30°C to an OD₆₀₀ of 0.7 and then shifted to 42°C for 2.0 hours. The proteins produced by these cells were analyzed by western blot analysis for protein band reactive with DCP-c₂a₂ peptide specific antisera. See Table 5 for results.

**TABLE 5**

| DCP-2 Expression clones | # of repeats | Gene size (in bp) | # of amino acids | Protein band observed (in kD) |
|---|---|---|---|---|
| pPT 0155 | 1 | 603 | 201 | no detection |
| pPT 0156 | 2 | 1035 | 345 | 40 |
| pPT 0157 | 3 | 1467 | 489 | 60 |
| pPT 0158 | 4 | 1899 | 633 | 80 |
| pPT 0159 | 5 | 2331 | 777 | 100 |
| pPT 0160 | 6 | 2763 | 921 | smear |
| pPT 0161 | 7 | 3195 | 1065 | no detection |
| pPT 0162 | 7 + | 3240 | 1080 | no detection |
| pPT 0163 | 7 + | 3420 | 1140 | no detection |

DCP-2    pPT 0159    777 Amino Acids    MW: 64,094 Dalton

Met-Asp-Pro-Val-Val-Leu-Gln-Arg-Arg-Asp-Trp-Glu-Asn-Pro-Gly-Val-Thr-
Gln-Leu-Asn-Arg-Leu-Ala-Ala-His-Pro-Pro-Phe-Ala-Ser-Asp-Pro-Met

[(Gly-Ala-His-Gly-Pro-Ala-Gly-Pro-Lys)2
(Gly-Ala-Pro-Gly-Pro-Ala-Gly-Pro-Pro)12
(Gly-Ala-His-Gly-Pro-Ala-Gly-Pro-Lys)2]5

Gly-Ala-Met-Asp-Pro-Gly-Arg-Tyr-Gln-Leu-Ser-Ala-Gly-Arg-Tyr-His-Tyr-
Gln-Leu-Val-Trp-Cys-Gln-Lys

### DCP3 MONOMER CONSTRUCTION

Four oligonucleotide strands encoding (ab)₂ were synthesized and purified as described (See, Example 1).

4.A) 5'- GTGCTCCGGGACCTGCAGAATATTATTCTAGAGGTGACCCAGGACCGCCTG - 3'
4.B) 3'- AGGCCCTGGACGTCTTATAATAAGATCTCCACTGGGTCCTGGCGGACCACG -5'
4.C) 5'- GGCCCACCGGGTAGCCGTGGCGATCCGGGACCACCGG-
4.D) 3'- ACGTCCGGGTGGCCCATCGGCACCGCTAGGCCCTGGTGGCC-

GTGCACCTGGCCCAGCGGGTCCGCCTGGAT - 3'
CACGTGGACCGGGTCGCCCAGGCGGACCTAGATC - 5'

Oligonucleotide strands 4.A and 4.B were annealed and ligatad with the DNA plasmid pPT 0134 (see example 1) which had been digested with FokI REN. The products of this ligation were transformed into E.coli strain HB101. Plasmid DNA from transformed colonies was purified and digested with PstI and StuI. Clones containing a fragment of approximately 800 bp were sequenced. Plasmid pPT 0139, containing the desired sequence of strands 4.A and 4.B, was chosen for subsequent constructions.

Strand 4.C and 4.D were annealed and ligated with the DNA plasmid pPT 0139 which had been previously digested with XbaI and PstI RENs. The products of this ligation were transformed into E.coli strain HB101. Plasmid DNA from transformed colonies was purified and digested with and NcoI and Dra III RENs. Clones containing a DNA fragment corresponding to the combined insertion of strands 4.A and B and 4.C and D were sequenced. Plasmid pPT0143, containing the correct (ab)₂ DNA sequence, as shown in Table 6, was chosen for further constructions.

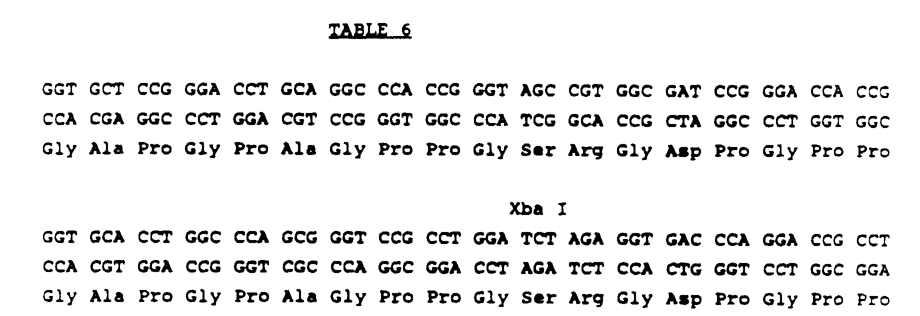

Plasmid DNA from pPT 0143 was digested with FokI REN and the fragment containing the (ab)₂ gene fragment was isolated by agarose gel electrophoresis and purified on a NACS column. The DNA fragment was then ligated with pPT 0134 that had been digested with FokI REN. The products of the ligation were transformed into E.coli strain HB101 and selected for growth on bacterial plates containing the antibiotic chloramphenicol. Plasmid DNA from individual colonies was analyzed for inserts containing multiple (ab)₂ DNA fragments by digestion with FokI REN. Several clones were obtained ranging from one copy to several copies of (ab)₂. One clone, pPT 0169, containing (ab)₆ was used as an intermediate for the construction of the DCP-3 gene monomer.

The (ab)₆ gene fragment Vam purified from pPT 0169 by digestion with FokI REN, agarose gel electrophoresis and NACS purification. This DNA fragment was then ligated with DNA plasmid pPT 0140 that had been previously digested with BanI REN, as illustrated in Figure 2. The products of the ligation were transformed into E.coli strain HB101, and transformants were selected on bacterial plates containing the antibiotic chloramphenicol. Plasmid DNA from individual colonies was analyzed by digestion with FokI REN for inserts containing multiple copies of the (ab)₆ gene fragment. One clone, pPT 0171, containing c₂(ab)₁₂c₂ (shown in Table 7) was chosen as the DCP-3 gene monomer for subsequent constructions.

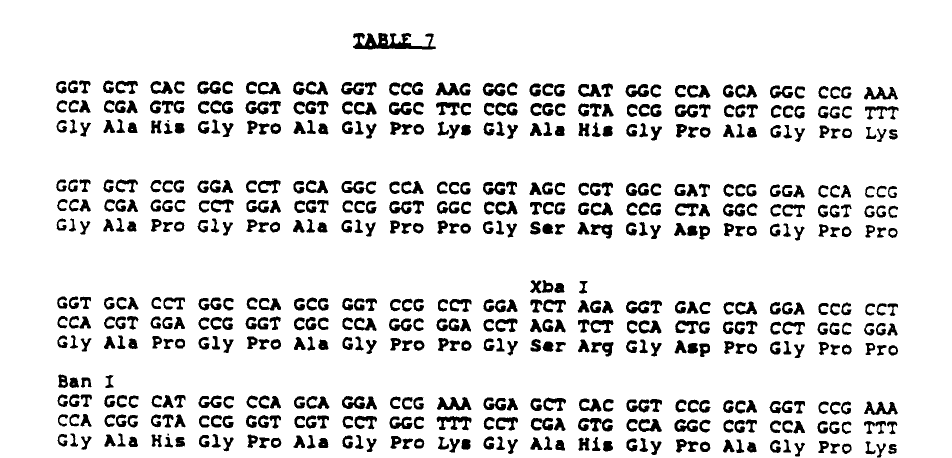

### DCP3 POLYMER GENE CONSTRUCTION

Plasmid DNA from pPT 0171 was digested with FokI REN and the fragment containing the DCP-3 gene monomer purified by agarose gel electrophoresis and NACS purification. The DCP-3 monomer was then self-ligated and then ligated with DNA plasmid pSY 1262 that had been digested with BanI REN, as illustrated in Figure 6. The products of the ligation were transformed into E.coli strain HB101 and selected for growth on bacterial plates containing the antibiotic kanamycin. Plasmid DNA from individual colonies was purified and analyzed after digestion with XcmI and PvuII RENs for insertions containing multiple copies of the DCP-3 gene monomer fragment. Clones pPT 0173, pPT 0174, pPT 0175 and pPT0176 containing monomer, dimer, trimer and tetramer forms of DCP-3, respectively, were selected for expression analysis.

### DCP-3 PROTEIN EXPRESSION ANALYSIS

E.coli strain HB101 containing DCP-3 plasmids pPT0173, pPT 0174, pPT 0175, or pPT 0176 were grown at 30° C to an OD₆₀₀ of 0.7 and then shifted to 42° C for 2.0 hours. The proteins produced by these cells were analyzed by western blot analysis for novel protein bands reactive with DCP peptide specific antisera. Reactive bands were observed with each clone (see Table 8 for results). However, the expression of the full length polymer decreased with the increased size of the genes. Northern analysis showed that the synthesis of full length mRNA in these clones was at equivalent levels. The probe for this northern analysis was the DCP-3 monomer fragment.

**TABLE 8**

| DCP-2 Expression clones observed (in kD) | # of repeats # of repeats | Gene size (in bp) | # of amino acids | Protein band observed (in kD) |
|---|---|---|---|---|
| pPT 0173 | 1 | 927 | 309 | 28 |
| pPT 0174 | 2 | 1683 | 561 | 64 |
| pPT 0175 | 3 | 2439 | 813 | 98 |
| pPT 0176 | 4 | 3195 | 1065 | 135 |

DCP3          pPT 0176          1065 Amino Acids  MW:   91,966 Dalton

Met-Asp-Pro-Val-Val-Leu-Gln-Arg-Arg-Asp-Trp-Glu-Asn-Pro-Gly-Val-Thr-
Gln-Leu-Asn-Arg-Leu-Ala-Ala-His-Pro-Pro-Phe-Ala-Ser-Asp-Pro-Met

((Gly-Ala-His-Gly-Pro-Ala-Gly-Pro-Lys)2
[(Gly-Ala-Pro-Gly-Pro-Ala-Gly-Pro-Pro)
(Gly-Ser-Arg-Gly-Asp-Pro-Gly-Pro-Pro)]12
(Gly-Ala-His-Gly-Pro-Ala-Gly-Pro-Lys)2)4

Gly-Ala-Met-Asp-Pro-Gly-Arg-Tyr-Gln-Leu-Ser-Ala-Gly-Arg-Tyr-His-Tyr-
Gln-Leu-Val-Trp-Cys-Gln-Lys

### EXAMPLE 3

**TABLE 9**

| STRUCTURE DCP-4, 5, & 6 | |
|---|---|
| DCP-4 | [c₂(db)₁₂ c₂]₄ |
| DCP-5 | [c₂(db)₆ c₂]₈ |
| DCP-6 | [C₂ d₂₄ c₂]₄ |

where:

b = Gly-Ser-Arg-Gly-Asp-Pro-Gly-Pro-Pro
c = Gly-Ala-His-Gly-Pro-Ala-Gly-Pro-Lys
d = Gly-Ala-Gln-Gly-Pro-Ala-Gly-Pro-Gly

### DCP-4 AND 5 MONOMER CONSTRUCTION

Three double stranded DNA sections, coding for (db)₃, were synthesized, purified and annealed as described in Example 1.

Eco01091
5.A) 5' - GTGCACAGGGACCGGCGGGACCAGGTGGCTCTCGAGGCGATCCGGGTCCTCCGG
5.B) 3' - TGTCCCTGGCCGCCCTGGTCCACCGAGAGCTCCGCTAGGCCCAGGAGGCCCACG
Eco01091 DraIII
5.C) 5' - GTGCACAAGGACCGGCAGGCCCTGGTGGCAGCCGCGGTGATCCGGGCCCACCGG
5.D) 3' - TGTTCCTGGCCGTCCGGGACCACCGTCGGCGCCACTAGGCCCGGGTGGCCCACG

5.E) 5' - GTGCTCAAGGACCGGCTGGCCCAGGCGGTTCCCGTGGAGACCCGGGTCCACCGG
5.F) 3' - AGTTCCTGGCCGACCGGGTCCGCCAAGGGCACCTCTGGGCCCAGGTGGCCCACG

The three DNA sections were cloned separately. The first two strand section (5.A & 5.B) was ligated to pPT 0138 previously digested with BanI REN. The ligation products were transformed into E.coli strain HB101 and selected for growth on bacterial plates containing the antibiotic chloramphenicol. Plasmid DNA from individual colonies was digested with EcoO1091 and StuI RENs and analyzed by agarose gel electrophoresis. Plasmid DNA containing an appropriately sized DNA fragment was sequenced and one clone, pPT 0221, was used in subsequent constructions.

The second pair of oligonucleotide strands (5.C & 5.D) were ligated with pPT 0134 which had been digested with FokI REN. After transformation of E.coli, plasmid DNA from individual colonies was analyzed by digestion with BanII and StuI RENs. DNAs that were digested by both enzymes were sequenced. One clone, pPT0222, had the expected sequence and was used for subsequent constructions.

Plasmid DNA from pPT 0222 was digested with FokI REN and the fragment (54bp) containing the second pair of oligonucleotide strands (5C & 5D was isolated by agarose gel electrophoresis followed by NACS purification. This DNA fragment was ligated with pPT 0221 previously digested with BanI REN. The products of the ligation were transformed into E.coli and plasmid DNA from single colonies was analyzed by agarose gel electrophoresis after digestion with DraIII and StuI RENs. One clone, pPT 0223, was chosen, after DNA sequencing, to be the acceptor vector for the third synthesized DCP gene fragment.

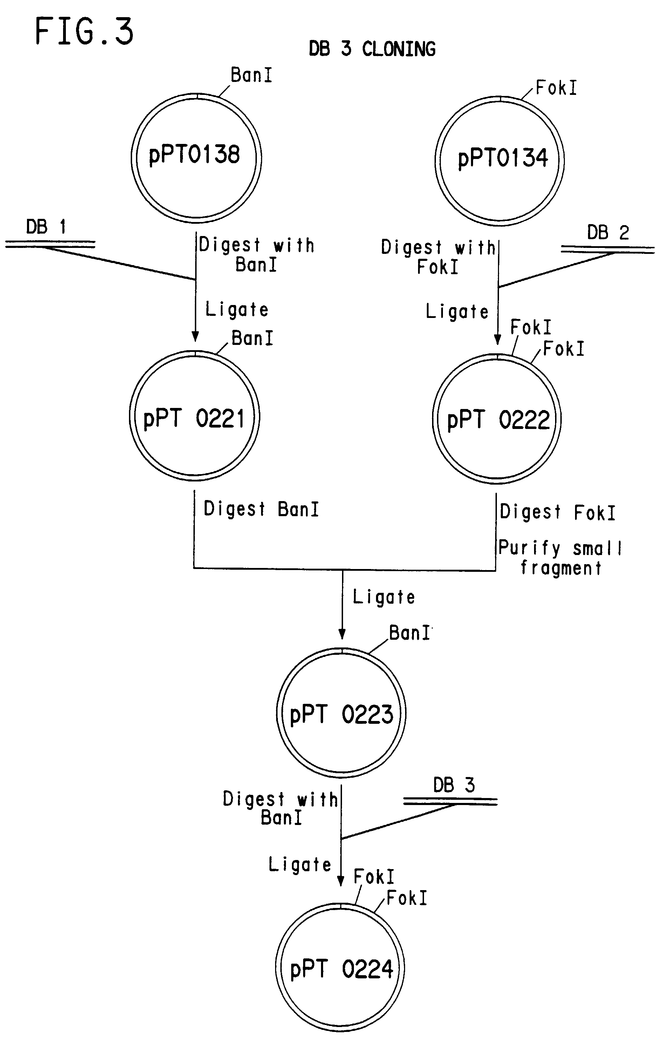

Plasmid pPT 0223 was digested with BanI REN and ligated with the third pair of oligonucleotide strands (5.E & 5.F). The product of the ligation reactions were transformed into E.coli strain HB101 and selected for growth on bacterial plates containing the antibiotic chloramphenicol. Plasmid DNA from individual transformants was purified and digested with FokI and BanI RENs. Clones containing the correct fragment size were sequenced. One clone, pPT 0224, shown in Table 10, containing the desired sequence was used in the subsequent constructions of the DCP-4 and DCP-5 monomers. The construction of pPT 0224 is illustrated in Figur. 3.

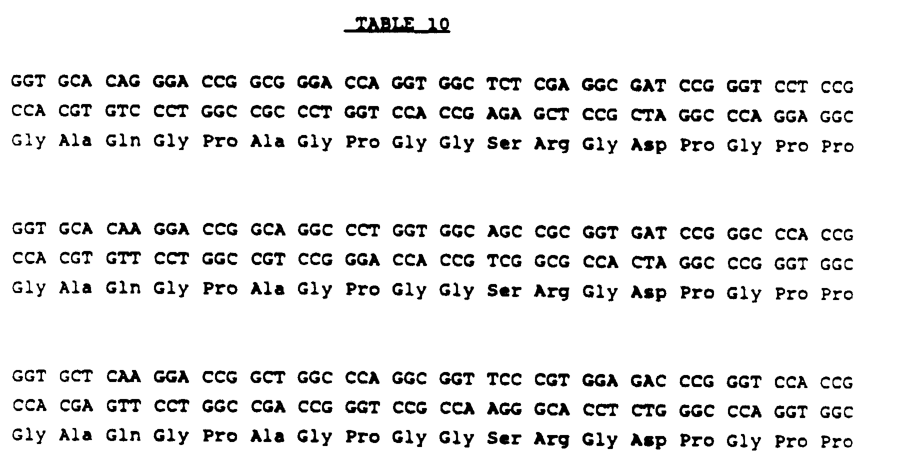

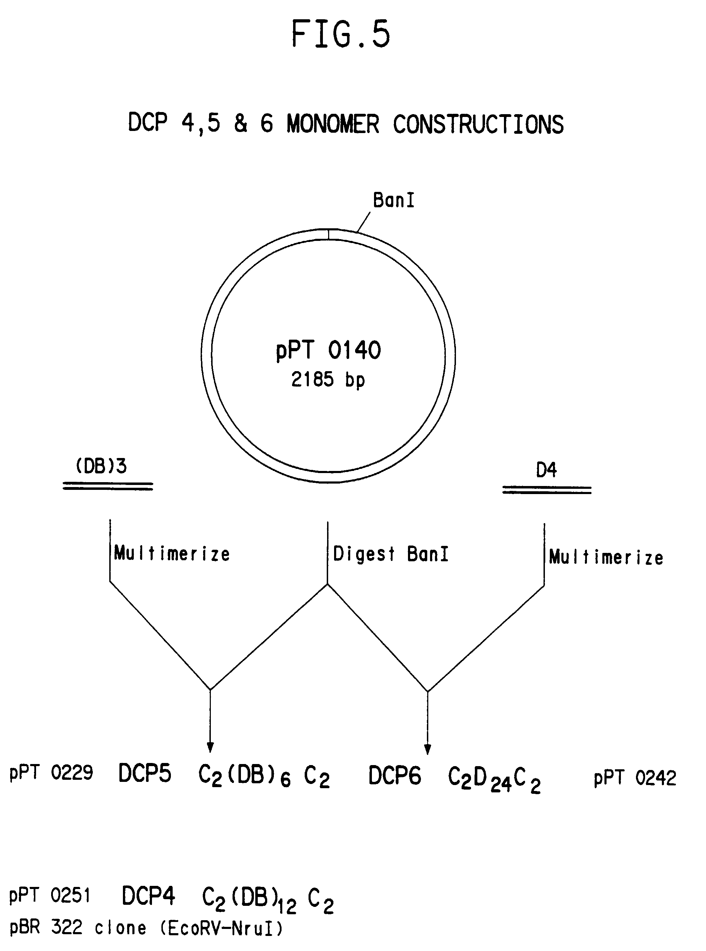

Plasmid DNA from pPT 0224 was digested with FokI and BanI RENs and the digestion fragment carrying (db)₃ was isolated by agarose gel electrophoresis and purified on a NACS column. The purified fragment was self-ligated and then cloned into pPT 0140 which had been digested with BanI REN, as illustrated in Figure 5. The products of the ligation were transformed into E.coli strain HB101. Plasmid DNA from individual colonies was analyzed for inserts containing multiple (db)₃ DNA fragments by digestion with FokI, Several size inserts were found ranging from (db)₃ to (db)₁₂. One clone, pPT 0229, was identified to contain the desired DCP-5 monomer sequence, c₂(db)₆c₂, and was used for subsequent constructions. The clone containing the DCP-4 gene monomer sequence c₂(db)₁₂c₂ was found to be highly unstable during subsequent passages, as was observed during the construction of DCP-1. Subsequently, the DCP-4 gene monomer was cloned into pBR 322,

The plasmid DNA containing the DCP-4 gene monomer fragment was isolated from the plasmid by digestion with NruI and EcoRV RENS, agarose gel electrophoresis, and purified on a NACS column. This DNA fragment was ligated with plasmid pBR 322 previously digested with EcoRV and NruI RENs (see agarose ligation in Example 1). The products of this ligation were transformed into E.coli strain HB101 and selected on bacterial plates containing ampicillin at 100 ug/ml. Plasmid DNA from individual colonies was analyzed by digestion with Scal REN. Clones containing the insert were further analyzed and one, pPT 0251, was chosen for the construction of the DCP-4 polymer gene. This plasmid was stable.

### DCP-4 POLYMER GENE CONSTRUCTION

Plasmid DNA from pPT 0251 was digested with AcyI REN and subsequently with FokI REN. The DNA fragment containing the DCP-4 gene monomer was isolated by agarose gel electrophoresis and purified using a NACS column (see Example 1). As illustrated in Figure 6, the monomer gene fragment was then ligated with pSY 1262 which had been previously digested with BanI REN, treated with phosphatase, and purified by gel electrophoresis and NACS column. The ligation products were transformed into E.coli strain HB101 and the transformants were selected for growth on bacterial plates containing the antibiotic kanamycin at 50 ug/ml. Plasmid DNA from individual colonies was purified and analyzed for multiple insertion of the DCP-4 gene monomer fragment. Several clones were obtained containing one, two or four copies of the DCP-4 gene monomer. Plasmids pPT0247, pPT0248 and pT0249 containing, respectively, 1, 2 and 4 copies of the gene monomer, were selected for protein expression analysis.

### DCP-4 PROTEIN EXPRESSION ANALYSIS

E.coli strain HB101 containing plasmids pPT 0247, pPT 0248, and pPT 0249 were grown at 30°C to an OD₆₀₀ of 0.7 and then shifted to 42° for 2.0 hours. The proteins produced by these cells were analyzed by western blot analysis for novel protein bands reactive with DCP-c₂a₂ peptide specific antisera. A reactive band corresponding to full length product was observed with each clone.

DCP-4    pPT0249   1,065 Amino Acids   MW: 91,533 Dalton

Met-Asp-Pro-Val-Val-Leu-Gln-Arg-Arg-Asp-Trp-Glu-Asn-Pro-Gly-Val-Thr-
Gln-Leu-Asn-Arg-Leu-Ala-Ala-His-Pro-Pro-Phe-Ala-Ser-Asp-Pro-Met

[(Gly-Ala-His-Gly-Pro-Ala-Gly-Pro-Lys)2[(Gly-Ala-Gln-Gly-Pro-Ala-Gly-
Pro-Gly)(Gly-Ser-Arg-Gly-Asp-Pro-Gly-Pro-Pro)]12(Gly-Ala-His-Gly-Pro-
Ala-Gly-Pro-Lys)2]4

Gly-Ala-Met-Asp-Pro-Gly-Arg-Tyr-Gln-Leu-Ser-Ala-Gly-Arg-Tyr-His-Tyr-
Gln-Leu-Val-Trp-Cys-Gln-Lys

### DCP-5 POLYMER GENE CONSTRUCTION

Plasmid DNA from pPT 0229 containing the DCP-5 gene monomer was isolated after digestion with FokI REN by agarose gel electrophoresis followed by NACS purification. As illustrated in Figure 6, the gene fragment was ligated with pSY 1262 which had been digested with BanI REN. The products of the ligation were transformed into E.coli strain HB101 and selected for growth on bacterial plates containing the antibiotic kanamycin at 50 ug/ml. Plasmid DNA from individual colonies was purified and analyzed for inserts containing multiple copies of the DCP-5 gene monomer sequence c₂(db)₆c₂. Plasmids pPT 0231 and pPT 0232, respectively, contained three and four copies of the DCP-5 gene monomer and were used for expression analysis.

### DCP-5 PROTEIN EXPRESSION ANALYSIS

E.coli strain HB101 containing plasmids pPT 0231 and pPT 0232 were grown at 30°C to an OD₆₀₀ of 0.7 and then shifted to 42°C for 2.0 hours. The proteins produced by these cells were analyzed by western blot analysis for novel protein bands reactive with DCP-c₂a₂ peptide specific antisera. A reactive band corresponding to full length product was observed with each clone.

DCP-5 pPT 0232 633 Amino Acids MW: 55,228 Daltons

Met-Asp-Pro-Val-Val-Leu-Gln-Arg-Arg-Asp-Trp-Glu-Asn-Pro-Gly-Val-Thr-
Gln-Leu-Asn-Arg-Leu-Ala-Ala-His-Pro-Pro-Phe-Ala-Ser-Asp-Pro-Met

[(Gly-Ala-His-Gly-Pro-Ala-Gly-Pro-Lys)2[(Gly-Ala-Gln-Gly-Pro-Ala-Gly-
Pro-Gly)(Gly-Ser-Arg-Gly-Asp-Pro-Gly-Pro-Pro)]6(Gly-Ala-His-Gly-Pro-
Ala-Gly-Pro-Lys)2]8

Gly-Ala-Met-Asp-Pro-Gly-Arg-Tyr-Gln-Leu-Ser-Ala-Gly-Arg-Tyr-His-Tyr-
Gln-Leu-Val-Trp-Cys-Gln-Lys

### DCP-6 GENE MONOMER CONSTRUCTION.

Four oligonucleotide strands encoding d4, were synthesized, purified and annealed as described in example 1.

EcoO 1091
6.A) 5' - GTGCACAGGGACCGGCGGGTCCAGGCGGTGCTCAAGGACCGGCAGGCCCTTAATTAAG
6.B) 3' -     TGTCCCTGGCCGCCCAGGTCCGCCACGAGTTCCTGGCCGTCCGGGAATTAATTCCACG
(EcoO 1091)
6.C) 5'-GCCCTGGTGGCGCTCAAGGTCCGGCTGGCCCAGGAGGCGCGCAGGGTCCGGCAGGTCCGGGAG
6.D )3'-    GACCACCGCGAGTTCCAGGCCGACCGGGTCCTCCGCGCGTCCCAGGCCGTCCAGGCCCTCCA

The first oligonucleotide segment consisting of strands 6A & 6B was ligated to pPT 0138 previously digested with BanI REN. The ligation products were transformed into E.coli strain HB101 and selected for grovth on bacterial plates containing the antibiotic chloramphenicol. Plasmid DNA from individual colonies was digested with EcoO1091 and XmnI RENs and analyzed by agarose gel electrophoresis. Plasmid DNA containing the correct sized fragment was sequenced and one clone, pPT 0219, containing the correct sequence was used for subsequent constructions.

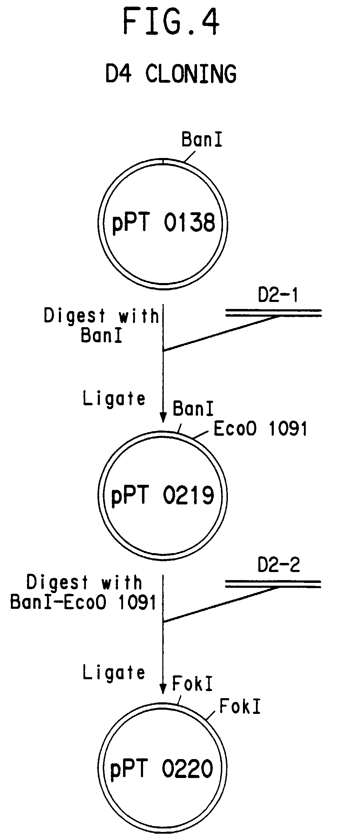

Plasmid pPT 0219 was digested with BanI REN and ligated with the second pair of oligonucleotide strands (6C & 6D). The products of the ligation were transformed into E.coli strain HB101 and selected for growth on bacterial plates containing the antibiotic chloramphenicol. Plasmid DNA from transformants was purified and digested with FokI and BanI RENs. Clones containing the correct fragment size were sequenced. One clone, pPT 0220, containing the sequence shown in Table 11 was used in subsequent constructions of the DCP-6 gene monomer. The construction of pPT 0220 is illustrated in Figure 4.

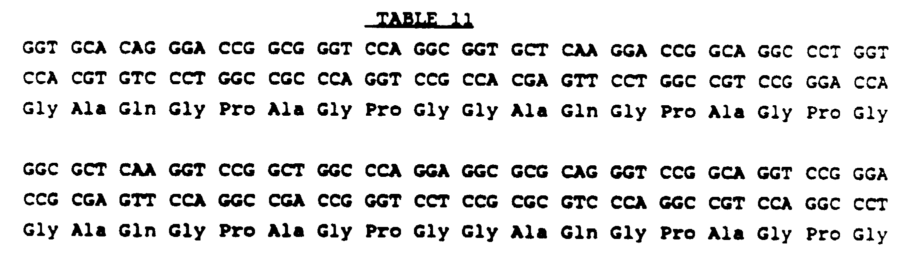

Plasmid DNA containing the d₄ sequence was digested with FokI and BanI REN. and the digestion fragment containing d₄ was isolated by agarose gel electrophoresis followed by NACS purification. As illustrated in Figure 5, the purified fragment was self-ligated and ligated with DNA plasmid pPT 0140 which had been digested with BanI REN. The ligation products were transformed into E.coli strain HB101 and selected for growth on bacterial plates containing the antibiotic chloramphenicol. The transformant, pPT 0242, containing the DCP-6 gene monomer ((d₄)₆ flanked by c₂-c₂) was used for subsequent constructions.

### DCP-6 POLYMER GENE CONSTRUCTION

Plasmid DNA from pPT 0242 was digested with FokI REN. The DNA fragment containing the DCP-6 gene monomer was isolated by agarose gel electrophoresis and purified using a NACS column. As illustrated in Figure 6, the monomer gene fragment was then ligated with pSY 1262 which had been previously digested with BanI REN, treated with phosphatase, and purified by gel electrophoresis and NACS column. The ligation products were transformed into E.coli strain HB101 and the transformants were selected for growth on bacterial plates containing the antibiotic kanamycin at 60 ug/ml. Plasmid DNA from individual colonies was purified and analyzed for multiple insertion of the DCP-6 gene monomer sequence, c₂d₂₄c₂. Several clones were obtained ranging from one to four repeats of the DCP-6 gene monomer. Plasmids pPT 0243, pPT 0244, pPT 0245, and pPT 0246 containing, respectively, 1, 2, 3 and 4 repeats of the gene monomer were selected for protein expression analysis.

### DCP-6 PROTEIN EXPRESSION ANALYSIS

E.coli strain HB101 containing plasmids pPT 0243 to pPT 0246 were grown at 30°C to an OD₆₀₀ of 0.7 and then shifted to 42°C for 2.0 hours. The proteins produced by these cells were analyzed by western blot analysis for novel protein bands reactive with DCP-c₂a₂ peptide specific anisera. See TABLE 11(a) for the results.

**TABLE 11(a)**

| DCP-6 Expression Clones | # of repeats | Gene size (in bp) | # of amino acids | Protein band observed (in kD) |
|---|---|---|---|---|
| pPT0243 | 1 | 927 | 309 | no detection |
| pPT0244 | 2 | 1683 | 561 | 72 |
| pPT0245 | 3 | 2439 | 813 | 110 |
| pPT0246 | 4 | 3195 | 1065 | 140 |

DCP-6 pPT0246 1,065 Amino Acids MW: 85,386 Daltons

Met-Asp-Pro-Val-Val-Leu-Gln-Arg-Arg-Asp-Trp-Glu-Asn-Pro-Gly-Val-Thr-
Gln-Leu-Asn-Arg-Leu-Ala-Ala-His-Pro-Pro-Phe-Ala-Ser-Asp-Pro-Met

[(Gly-Ala-His-Gly-Pro-Ala-Gly-Pro-Lys)2[(Gly-Ala-Gln-Gly-Pro-Ala-Gly-
Pro-Gly)24(Gly-Ala-His-Gly-Pro-Ala-Gly-Pro-Lys)2]4

Gly-Ala-Met-Asp-Pro-Gly-Arg-Tyr-Gln-Leu-Ser-Ala-Gly-Arg-Tyr-His-Tyr-
Gln-Leu-Val-Trp-Cys-Gln-Lys

**TABLE 12**

| Amino Acid | One-Letter Symbol | Three-Letter Symbol |
|---|---|---|
| Alanine | A | Ala |
| Arginine | R | Arg |
| Asparagine | N | Asn |
| Aspartic Acid | D | Asp |
| Asparagine and/or | | |
| Aspartic Acid | B | Asx |
| Cysteine | C | Cys |
| Glutamine | Q | Gln |
| Glutamic Acid | E | Glu |
| Glutamine and/or | | |
| Glutamic Acid | Z | Glx |
| Glycine | G | Gly |
| Histidine | H | His |
| Isoleucine | I | Ile |
| Leucine | L | Leu |
| Lysine | K | Lys |
| Methionine | M | Met |
| Phenylalanine | F | Phe |
| Proline | P | Pro |
| Serine | S | Ser |
| Threonine | T | Thr |
| Tryptophan | W | Trp |
| Tyrosine | Y | Tyr |
| Valine | V | Val |
| Hydroxyproline | | Hyp |

## Claims

1. A collagen-like block copolymer, containing at least 250 amino acids and characterized by the formula:
[C_{f1} (Aⱼ₁ Bⱼ₂)ₖ C_{f2}]ₘ
where,
A = a block peptide unit having amino acid sequences of the formula, [Gly - x - y]₃₋₃₀, wherein about 50 to 75% of the amino acids, x and y, are each independently selected from the group consisting of proline and hydroxyproline, each amino acid sequence, [Gly - x - y], being the same or different;
B = a block peptide unit having amino acid sequences of the formula, [Gly - x - y]₃₋₃₀, wherein about 25 to 60% of the amino acids, x and y, are each independently selected from the group consisting of proline and hydroxyproline, each amino acid sequence, [Gly - x - y], being the same or different;
C = a block peptide unit having amino acid sequences of the formula, [Gly - x - y]₃₋₃₀, wherein about 25 to 60% of the amino acids, x and y, are each independently selected from the group consisting of proline and hydroxyproline, and at least one of the remaining amino acids, x and y, is independently selected from the group consisting of lysine, arginine, histidine, cysteine, tyrosine, aspartic acid, and glutamic acid, each amino acid sequence, [Gly - x - y], being the same or different;
f1, k, f2, and m are each equal to or greater than one; and
the sum of j1 and j2 is equal to or greater than one.

2. A collagen-like polypeptide, characterized by having amino acid sequences of the formula, [Gly - x - y]₃₋₃₀, wherein about 25 to 60% of the amino acids, x and y, are each independently selected from the group consisting of proline and hydroxyproline, and at least one of the remaining amino acids, x and y, is independently selected from the group consisting of lysine, arginine, histidine, cysteine, tyrosine, aspartic acid, and glutamic acid, each amino acid sequence, [Gly - x - y], being the same or different.

3. A collagen-like block copolymer, containing at least 250 amino acids and characterized by the formula:
[C_{f1} (Aⱼ₁ Bⱼ₂)ₖ C_{f2}]ₘ
where,
A = a block peptide unit having amino acid sequences of the formula, [Gly - x - y]₃₋₃₀, wherein about 50 to 75% of the amino acids, x and y, are each independently selected from the group consisting of proline and hydroxyproline, each amino acid sequence, [Gly - x - y], being the same or different;
B = a block peptide unit having amino acid sequences of the formula, [Gly - x - y]₃₋₃₀, wherein about 25 to 60% of the amino acids, x and y, are each independently selected from the group consisting of proline and hydroxyproline, each amino acid sequence, [Gly - x - y], being the same or different;
C = a block peptide unit having amino acid sequences of the formula, [Gly - x - y]₃₋₃₀, wherein about 25 to 60% of the amino acids, x and y, are each independently selected from the group consisting of proline and hydroxyproline, and at least one of the remaining amino acids, x and y, is independently selected from the group consisting of lysine, arginine, histidine, cysteine, tyrosine, aspartic acid, and glutanic acid, each amino acid sequence, [Gly - x - y], being the same or different;
k and m are each equal to or greater than one;
the sum of f1 and f2 is equal to or greater than one; and
the sum of j1 and j2 is equal to or greater than one.

4. The collagen-like block copolymer of claim 1, where all of the amino acids, x and y, which are not proline or hydroxyproline in block unit A are each independently selected from the group consisting of glycine, alanine, and serine.

5. The collagen-like block copolymer of claim 4, where block unit A comprises the amino acid sequence:

Gly-Ala-Pro-Gly-Pro-Ala-Gly-Pro-Pro.

6. The collagen-like block copolymer of claim 1, where at least about 66% of the amino acids, x and y, in block unit B are each independently selected from the group consisting of proline, hydroxyproline, glycine, alanine, and serine.

7. The collagen-like block copolymer of claim 6, where block unit B comprises the amino acid sequence:

Gly-Ser-Arg-Gly-Asp-Pro-Gly-Pro-Pro.

8. The collagen-like block copolymer of claim 6, where block unit B comprises the amino acid sequence:

Gly-Ala-Gln-Gly-Pro-Ala-Gly-Pro-Gly.

9. The collagen-like block copolymer of claim 1, where block unit B comprises an amino acid sequence selected from the group consisting of 
Gly-Gly-Arg-Gly-Asp-Ser-Gly-Pro-Pro

Gly-Pro-Pro-Gly-Gly-Arg-Gly-Asp-Ser

Gly-Tyr-Ile-Gly-Ser-Arg-Gly-Pro-Pro;
 and

Gly-Pro-Pro-Gly-Tyr-Ile-Gly-Ser-Arg.

10. The collagen-like block copolymer of claim 1, where at least about 50% of the amino acids, x and y, in block unit C, are each independently selected from the group consisting of proline, hydroxyproline, glycine, alanine, and serine.

11. The collagen-like block copolymer of claim 10, where at least one of the remaining amino acids, x and y, is independently selected from the group consisting of aspartic acid, glutamic acid, lysine, arginine, histidine, tyrosine, and cysteine.

12. The collagen-like block copolymer of claim 10, where at least one of the remaining amino acids, x and y, is independently selected from the group consisting of lysine, cysteine, tyrosine, aspartic acid, and glutamic acid.

13. The collagen-like block copolymer of claim 10, where at least one of the remaining amino acids, x and y, is independently selected from the group consisting of aspartic acid, glutamic acid, lysine, arginine, histidine, tyrosine, and cysteine and at least one of the remaining amino acids, x and y, is independently selected from the group consisting of lysine, cysteine, tyrosine, aspartic acid, and glutamic acid.

14. The collagen-like block copolymer of claim 1, where block unit C comprises the amino acid sequence:

Gly-Ala-His-Gly-Pro-Ala-Gly-Pro-Lys.

15. The collagen-like block copolymer of claim 1, where block unit C comprises an amino acid sequence selected from the group consisting of: 
Gly-Pro-Lys-Gly-Lys-Ala-Gly-Pro-Lys

Gly-Pro-His-Gly-Lys-Ala-Gly-Pro-Lys
 
Gly-Pro-His-Gly-Lys-Ala-Gly-Pro-His

Gly-Pro-His-Gly-His-Ala-Gly-Pro-His

Gly-Pro-Glu-Gly-His-Ala-Gly-Pro-Lys

Gly-Pro-Glu-Gly-His-Ala-Gly-Pro-His

Gly-Pro-Glu-Gly-His-Ala-Gly-Pro-Glu;
 and

Gly-Pro-Glu-Gly-Glu-Ala-Gly-Pro-Glu

16. The collagen-like block copolymer of claim 1, having the formula:
[ C₂ A₂₄ C₂ ]₁₋₈
where,
block unit A comprises the amino acid sequence, Gly-Ala-Pro-Gly-Pro-Ala-Gly-Pro-Pro, and
block unit C comprises the amino acid sequence,

Gly-Ala-His-Gly-Pro-Ala-Gly-Pro-Lys.

17. The collagen-like block copolymer of claim 16, having the formula:
[ C₂ A₂₄ C₂ ]₄.

18. The collagen-like block copolymer of claim 1, having the formula:
[ C₂ A₁₂ C₂ ]₂₋₁₆
where,
block unit A comprises the amino acid sequence, Gly-Ala-Pro-Gly-Pro-Ala-Gly-Pro-Pro, and
block unit C comprises the amino acid sequence,

Gly-Ala-His-Gly-Pro-Ala-Gly-Pro-Lys.

19. The collagen-like block copolymer of claim 18, having the formula:
[ C₂ A₁₂ C₂ ]₈.

20. The collagen-like block copolymer of claim 1, having the formula:
[ C₂ (A B)₁₂ C₂ ]₁₋₈
where,
block unit A comprises the amino acid sequence, Gly-Ala-Pro-Gly-Pro-Ala-Gly-Pro-Pro,
block unit B comprises the amino acid sequence, Gly-Ser-Arg-Gly-Asp-Pro-Gly-Pro-Pro, and
block unit C comprises the amino acid sequence,

Gly-Ala-His-Gly-Pro-Ala-Gly-Pro-Lys.

21. The collagen-like block copolymer of claim 20, having the formula:
[ C₂ (A B)₁₂ C₂ ]₄.

22. The collagen-like block copolymer of claim 1, having the formula:
[ C₂ B₁₂ C₂ ]₁₋₈
where,
block unit B comprises the amino acid sequence, 
Gly-Ala-Gln-Gly-Pro-Ala-Gly-Pro-Gly-
Gly-Ser-Arg-Gly-Asp-Pro-Gly-Pro-Pro;
 and
block unit C comprises the amino acid sequence,

Gly-Ala-His-Gly-Pro-Ala-Gly-Pro-Lys.

23. The collagen-like block copolymer of claim 22, having the formula:
[ C₂ B₁₂ C₂ ]₄.

24. The collagen-like block copolymer of claim 1, having the formula:
[ C₂ B₆ C₂ ]₂₋₁₆
where,
block unit B comprises the amino acid sequence, 
Gly-Ala-Gln-Gly-Pro-Ala-Gly-Pro-Gly-
Gly-Ser-Arg-Gly-Asp-Pro-Gly-Pro-Pro;
 and
block unit C comprises the amino acid sequence,

Gly-Ala-His-Gly-Pro-Ala-Gly-Pro-Lys.

25. The collagen-like block copolymer of claim 24, having the formula:
[ C₂ B₆ C₂ ]₈.

26. The collagen-like block copolymer of claim 1, having the formula:
[ C₂ B₂₄ C₂ ]₁₋₈
where,
block unit B comprises the amino acid sequence, Gly-Ala-Gln-Gly-Pro-Ala-Gly-Pro-Gly, and
block unit C comprises the amino acid sequence,

Gly-Ala-His-Gly-Pro-Ala-Gly-Pro-Lys.

27. The collagen-like block copolymer of claim 26, having the formula:
[ C₂ B₂₄ C₂ ]₄.

28. A polymeric blend, comprising the collagen-like block copolymer of claim 1, 2 or 3 and natural collagen.

29. A fiber, comprising the collagen-like block copolymer of claim 1, 2 or 3.

30. A film, comprising the collagen-like block copolymer of claim 1, 2 or 3.

31. A coating, comprising the collagen-like block copolymer of claim 1, 2 or 3.

32. A medical implant, comprising the collagen-like block copolymer of claim 1, 2 or 3.

## Patentansprüche

1. Ein collagenartiges Blockcopolymer, welches mindestens 250 Aminosäuren enthält und gekennzeichnet ist durch die Formel:
[C_{f1} (Aⱼ₁ Bⱼ₂)ₖ C_{f2}]ₘ
wobei
A = eine Blockpeptideinheit mit Aminosäurensequenzen der Formel [Gly - x - y]₃₋₃₀ ist, wobei etwa 50 bis 75% der Aminosäuren, x und y, jeweils unabhängig aus der Gruppe aus Prolin und Hydroxyprolin ausgewählt sind, jede Aminosäurensequenz [Gly - x - y] die gleiche oder unterschiedlich ist;
B = eine Blockpeptideinheit mit Aminosäurensequenzen der Formel [Gly - x - y]₃₋₃₀ ist, wobei etwa 25 bis 60% der Aminosäuren, x und y jeweils unabhängig ausgewählt sind aus der Gruppe aus Prolin und Hydroxyprolin, jede Aminosäurensequenz [Gly - x - y] die gleiche oder unterschiedlich ist;
C = eine Blockpeptideinheit mit Aminosäurensequenzen der Formel [Gly - x - y]₃₋₃₀ ist, wobei etwa 25 bis 60% der Aminosäuren, x und y, jeweils unabhängig ausgewählt sind aus der Gruppe aus Prolin und Hydroxyprolin, und mindestens eine der verbleibenden Aminosäuren, x und y, unabhängig ausgewählt ist aus der Gruppe aus Lysin, Arginin, Histidin, Cystein, Tyrosin, Asparginsäure und Glutaminsäure, jede Aminosäurensequenz [Gly - x - y] die gleiche oder unterschiedlich ist;
f1, k, f2 und m jeweils gleich oder größer als eins sind; und
die Summe aus j1 und j2 gleich oder größer als eins ist.

2. Ein collagenartiges Polypeptid, dadurch gekennzeichnet, daß es Aminosäurensequenzen der Formel [Gly - x -y]₃₋₃₀ hat, wobei etwa 25 bis 60% der Aminosäuren, x und y, jeweils unabhängig ausgewählt sind aus der Gruppe aus Prolin und Hydroxyprolin, und mindestens eine der verbleibenden Aminosäuren, x und y, unabhängig ausgewählt ist aus der Gruppe aus Lysin, Arginin, Histidin, Cystein, Tyrosin, Asparginsäure und Glutaminsäure, jede Aminosäurensequenz [Gly - x - y] die gleiche oder unterschiedlich ist.

3. Ein collagenartiges Blockcopolymer, welches mindestens 250 Aminosäuren enthält und gekennzeichnet ist durch die Formel
[C_{f1} (Aⱼ₁ Bⱼ₂)ₖ C_{f2}]ₘ
wobei
A = eine Blockpeptideinheit mit Aminosäurensequenzen der Formel [Gly - x - y]₃₋₃₀ ist, wobei etwa 50 bis 75% der Aminosäuren, x und y, jeweils unabhängig ausgewählt sind aus der Gruppe aus Prolin und Hydroxyprolin, wobei jede Aminosäurensequenz [Gly - x - y] die gleiche oder unterschiedlich ist;
B = eine Blockpeptideinheit mit Aminosäurensequenzen der Formel [Gly - x - y]₃₋₃₀ ist, wobei etwa 25 bis 60% der Aminosäuren, x und y, jeweils unabhängig ausgewählt sind aus der Gruppe aus Prolin und Hydroxyprolin, wobei jede Aminosäurensequenz [Gly - x - y] die gleiche oder unterschiedlich ist;
C = eine Blockpeptideinheit mit Aminosäurensequenzen der Formel [Gly - x - y]₃₋₃₀ ist, wobei etwa 25 bis 60% der Aminosäuren, x und y, jeweils unabhängig ausgewählt sind aus der Gruppe aus Prolin und Hydroxyprolin, und mindestens eine der verbleibenden Aminosäuren, x und y, unabhängig ausgewählt ist aus der Gruppe aus Lysin, Arginin, Histidin, Cystein, Tyrosin, Asparginsäure und Glutaminsäure, wobei jede Aminosäurensequenz [Gly - x - y] die gleiche oder unterschiedlich ist;
k und m jeweils gleich oder größer als eins sind;
die Summe aus f1 und f2 gleich oder größer als eins ist; und
die Summe aus j1 und j2 gleich oder größer als eins ist.

4. Das collagenartige Blockcopolymer nach Anspruch 1, wobei alle die Aminosäuren, x und y,, die nicht Prolin oder Hydroxyprolin in der Blockeinheit A sind, jeweils unabhängig ausgewählt sind aus der Gruppe aus Glycin, Alanin und Serin.

5. Das collagenartige Blockcopolymer nach Anspruch 4, wobei die Blockeinheit A die Aminosäurensequenz: Gly-Ala-Pro-Gly-Pro-Ala-Gly-Pro-Pro umfaßt.

6. Das collagenartige Blockcopolymer nach Anspruch 1, wobei mindestens etwa 66% der Aminosäuren, x und y, in der Blockeinheit B jeweils unabhängig ausgewählt sind aus der Gruppe aus Prolin, Hydroxyprolin, Glycin, Alanin und Serin.

7. Das collagenartige Blockcopolymer nach Anspruch 6, wobei die Blockeinheit B die Aminosäurensequenz: Gly-Ser-Arg-Gly-Asp-Pro-Gly-Pro-Pro umfaßt.

8. Das collagenartige Blockcopolymer nach Anspruch 6, wobei die Blockeinheit B die Aminosäuresequenz: Gly-Ala-Gln-Gly-Pro-Ala-Gly-Pro-Gly umfaßt.

9. Das collagenartige Blockcopolymer nach Anspruch 1, wobei die Blockeinheit B eine Aminosäuresequenz ausgewählt aus der Gruppe aus 
Gly-Gly-Arg-Gly-Asp-Ser-Gly-Pro-Pro
Gly-Pro-Pro-Gly-Gly-Arg-Gly-Asp-Ser
Gly-Tyr-Ile-Gly-Ser-Arg-Gly-Pro-Pro;
 und Gly-Pro-Pro-Gly-Tyr-Ile-Gly-Ser-Arg umfaßt.

10. Das collagenartige Blockcopolymer nach Anspruch 1, wobei mindestens etwa 50% der Aminosäuren, x und y, in der Blockeinheit C jeweils unabhängig ausgewählt sind aus der Gruppe aus Prolin, Hydroxyprolin, Glycin, Alanin und Serin.

11. Das collagenartige Blockcopolymer nach Anspruch 10, wobei mindestens eine der verbleibenden Aminosäuren, x und y, unabhängig ausgewählt ist aus der Gruppe aus Asparginsäure, Glutaminsäure, Lysin, Arginin, Histidin, Tyrosin und Cystein.

12. Das collagenartige Blockcopolymer nach Anspruch 10, wobei mindestens eine der verbleibenden Aminosäuren, x und y, unabhängig ausgewählt ist aus der Gruppe aus Lysin, Cystein, Tyrosin, Asparginsäure und Glutaminsäure.

13. Das collagenartige Blockcopolymer nach Anspruch 10, wobei mindestens eine der verbleibenden Aminosäuren, x und y, unabhängig ausgewählt ist aus der Gruppe aus Asparginsäure, Glutaminsäure, Lysin, Arginin, Histidin, Tyrosin und Cystein, und mindestens eine der verbleibenden Aminosäuren, x und y, unabhängig ausgewählt ist aus der Gruppe aus Lysin, Cystein, Tyrosin, Asparginsäure und Glutaminsäure.

14. Das collagenartige Blockcopolymer nach Anspruch 1, wobei die Blockeinheit C die Aminosäurensequenz: Gly-Ala-His-Gly-Pro-Ala-Gly-Pro-Lys umfaßt.

15. Das collagenartige Blockcopolymer nach Anspruch 1, wobei die Blockeinheit C eine Aminosäurensequenz ausgewählt aus der Gruppe aus: 
Gly-Pro-Lys-Gly-Lys-Ala-Gly-Pro-Lys
Gly-Pro-His-Gly-Lys-Ala-Gly-Pro-Lys
Gly-Pro-His-Gly-Lys-Ala-Gly-Pro-His
Gly-Pro-His-Gly-His-Ala-Gly-Pro-His
Gly-Pro-Glu-Gly-His-Ala-Gly-Pro-Lys
Gly-Pro-Glu-Gly-His-Ala-Gly-Pro-His
Gly-Pro-Glu-Gly-His-Ala-Gly-Pro-Glu;
 und Gly-Pro-Glu-Gly-Glu-Ala-Gly-Pro-Glu umfaßt.

16. Das collagenartige Blockcopolymer nach Anspruch 1 mit der Formel
[C₂ A₂₄ C₂]₁₋₈
wobei
die Blockeinheit A die Aminosäurensenquenz Gly-Ala-Pro-Gly-Pro-Ala-Gly-Pro-Pro umfaßt, und
die Blockeinheit C die Aminosäurensequenz Gly-Ala-His-Gly-Pro-Ala-Gly-Pro-Lys umfaßt.

17. Das collagenartige Blockcopolymer nach Anspruch 16 mit der Formel
[C₂ A₂₄ C₂]₄.

18. Das collagenartige Blockcopolymer nach Anspruch 1 mit der Formel
[C₂ A₁₂ C₂]₂₋₁₆
wobei
die Blockeinheit A die Aminosäurensequenz Gly-Ala-Pro-Gly-Pro-Ala-Gly-Pro-Pro umfaßt, und
die Blockeinheit C die Aminosäurensequenz Gly-Ala-His-Gly-Pro-Ala-Gly-Pro-Lys umfaßt.

19. Das collagenartige Blockcopolymer nach Anspruch 18 mit der Formel:
[C₂ A₁₂ C₂]₈.

20. Das collagenartige Blockcopolymer nach Anspruch 1 mit der Formel
[C₂ (A B)₁₂ C₂]₁₋₈
wobei
die Blockeinheit A die Aminosäurensequenz Gly-Ala-Pro-Gly-Pro-Ala-Gly-Pro-Pro umfaßt,
die Blockeinheit B die Aminosäurensequenz Gly-Ser-Arg-Gly-Asp-Pro-Gly-Pro-Pro umfaßt, und
die Blockeinheit C die Aminosäurensequenz Gly-Ala-His-Gly-Pro-Ala-Gly-Pro-Lys umfaßt.

21. Das collagenartige Blockcopolymer nach Anspruch 20 mit der Formel:
[C₂ (A B)₁₂ C₂]₄.

22. Das collagenartige Blockcopolymer nach Anspruch 1 mit der Formel
[C₂ B₁₂ C₂]₁₋₈
wobei
die Blockeinheit B die Aminosäurensequenz 
Gly-Ala-Gln-Gly-Pro-Ala-Gly-Pro-Gly-
Gly-Ser-Arg-Gly-Asp-Pro-Gly-Pro-Pro
 umfaßt; und
die Blockeinheit C die Aminosäurensequenz Gly-Ala-His-Gly-Pro-Ala-Gly-Pro-Lys umfaßt.

23. Das collagenartige Blockcopolymer nach Anspruch 22 mit der Formel
[C₂ B₁₂ C₂]₄.

24. Das collagenartige Blockcopolymer nach Anspruch 1 mit der Formel:
[C₂ B₆ C₂]₂₋₁₆
wobei
die Blockeinheit B die Aminosäurensequenz 
Gly-Ala-Gln-Gly-Pro-Ala-Gly-Pro-Gly-
Gly-Ser-Arg-Gly-Asp-Pro-Gly-Pro-Pro
 umfaßt, und
die Blockeinheit C die Aminosäurensequenz Gly-Ala-His-Gly-Pro-Ala-Gly-Pro-Lys umfaßt.

25. Das collagenartige Blockcopolymer nach Anspruch 24 mit der Formel
[C₂ B₆ C₂]₈.

26. Das collagenartige Blockcopolymer nach Anspruch 1 mit der Formel:
[C₂ B₂₄ C₂]₁₋₈
wobei
die Blockeinheit B die Aminosäuresequenz Gly-Ala-Gln-Gly-Pro-Ala-Gly-Pro-Gly umfaßt, und
die Blockeinheit C die Aminosäurensequenz Gly-Ala-His-Gly-Pro-Ala-Gly-Pro-Lys umfaßt.

27. Das collagenartige Blockcopolymer nach Anspruch 26 mit der Formel
[C₂ B₂₄ C₂]₄.

28. Eine Polymermischung, welche das collagenartige Blockcopolymer nach Anspruch 1, 2 oder 3 und natürliches Collagen umfaßt.

29. Eine Faser, welche das collagenartige Blockcopolymer nach Anspruch 1, 2 oder 3 umfaßt.

30. Ein Film, welcher das collagenartige Blockcopolymer nach Anspruch 1, 2 oder 3 umfaßt.

31. Ein Überzug, welcher das collagenartige Blockcopolymer nach Anspruch 1, 2 oder 3 umfaßt.

32. Ein medizinisches Implantat, welches das collagenartige Blockcopolymer nach Anspruch 1, 2 oder 3 umfaßt.

## Revendications

1. Copolymère bloc analogue au collagène, contenant au moins 250 acides aminés et caractérisé par la formule:
[C_{f1} (Aⱼ₁ Bⱼ₂)ₖ C_{f2}]ₘ
où,
A = une unité peptidique bloc ayant des séquences d'acides aminés de formule [Gly - x - y]₃₋₃₀, dans laquelle environ 50 à 75% des acides aminés, x et y, sont chacun indépendamment choisis dans le groupe constitué de la proline et de l'hydroxyproline, chaque séquence d'acides aminés, [Gly - x - y], étant identique ou différente;
B = une unité peptidique bloc ayant des séquences d'acides aminés de formule [Gly - x - y]₃₋₃₀, dans laquelle environ 25 à 60% des acides aminés, x et y, sont chacun indépendamment choisis dans le groupe constitué de la proline et de l'hydroxyproline, chaque séquence d'acides aminés, [Gly - x - y], étant identique ou différente;
C = une unité peptidique bloc ayant des séquences d'acides aminés de formule [Gly - x - y]₃₋₃₀, dans laquelle environ 25 à 60% des acides aminés, x et y, sont chacun indépendamment choisis dans le groupe constitué de la proline et de l'hydroxyproline, et au moins un des acides aminés restants, x et y, est indépendamment choisi dans le groupe constitué de la lysine, de l'arginine, de l'histidine, de la cystéine, de la tyrosine, de l'acide aspartique, et de l'acide glutamique, chaque séquence d'acides aminés, [Gly - x - y], étant identique ou différente;
f1, k, f2, et m sont chacun supérieurs ou égaux à un; et
la somme de j1 et de j2 est supérieure ou égale à un.

2. Polypeptide analogue au collagène, caractérisé par le fait d'avoir des séquences d'acides aminés de formule [Gly - x - y]₃₋₃₀, dans laquelle environ 25 à 60% des acides aminés, x et y, sont chacun indépendamment choisis dans le groupe constitué de la proline et de l'hydroxyproline, et au moins un des acides aminés restants, x et y, est indépendamment choisi dans le groupe constitué de la lysine, de l'arginine, de l'histidine, de la cystéine, de la tyrosine, de l'acide aspartique, et de l'acide glutamique, chaque séquence d'acides aminés, [Gly - x - y], étant identique ou différente.

3. Copolymère bloc analogue au collagène, contenant au moins 250 acides aminés et caractérisé par la formule:
[C_{f1} (Aⱼ₁ Bⱼ₂)ₖ C_{f2}]ₘ
où,
A = une unité peptidique bloc ayant des séquences d'acides aminés de formule [Gly - x - y]₃₋₃₀, dans laquelle environ 50 à 75% des acides aminés, x et y, sont chacun indépendamment choisis dans le groupe constitué de la proline et de l'hydroxyproline, chaque séquence d'acides aminés, [Gly - x - y], étant identique ou différente;
B = une unité peptidique bloc ayant des séquences d'acides aminés de formule [Gly - x - y]₃₋₃₀, dans laquelle environ 25 à 60% des acides aminés, x et y, sont chacun indépendamment choisis dans le groupe constitué de la proline et de l'hydroxyproline, chaque séquence d'acides aminés, [Gly - x - y], étant identique ou différente;
C = une unité peptidique bloc ayant des séquences d'acides aminés de formule [Gly - x - y]₃₋₃₀, dans laquelle environ 25 à 60% des acides aminés, x et y, sont chacun indépendamment choisis dans le groupe constitué de la proline et de l'hydroxyproline, et au moins un des acides aminés restants, x et y, est indépendamment choisi dans le groupe constitué de la lysine, de l'arginine, de l'histidine, de la cystéine, de la tyrosine, de l'acide aspartique, et de l'acide glutamique, chaque séquence d'acides aminés, [Gly - x - y], étant identique ou différente;
k et m sont chacun supérieurs ou égaux à un;
la somme de f1 et de f2 est supérieure ou égale à un; et
la somme de j1 et de j2 est supérieure ou égale à un.

4. Copolymère bloc analogue au collagène selon la revendication 1, où tous les acides aminés, x et y, qui ne sont pas la proline ou l'hydroxyproline dans l'unité bloc A, sont chacun indépendamment choisis dans le groupe constitué de la glycine, de l'alanine et de la sérine.

5. Copolymère bloc analogue au collagène selon la revendication 4, où l'unité bloc A comprend la séquence d'acides aminés:

Gly-Ala-Pro-Gly-Pro-Ala-Gly-Pro-Pro.

6. Copolymère bloc analogue au collagène selon la revendication 1, où au moins 66% environ des acides aminés, x et y, dans l'unité bloc B sont chacun indépendamment choisis dans le groupe constitué de la proline, de l'hydroxyproline, de la glycine, de l'alanine et de la sérine.

7. Copolymère bloc analogue au collagène selon la revendication 6, où l'unité bloc B comprend la séquence d'acides aminés:

Gly-Ser-Arg-Gly-Asp-Pro-Gly-Pro-Pro.

8. Copolymère bloc analogue au collagène selon la revendication 6, où l'unité bloc B comprend la séquence d'acides aminés:

Gly-Ala-Gln-Gly-Pro-Ala-Gly-Pro-Gly.

9. Copolymère bloc analogue au collagène selon la revendication 1, où l'unité bloc B comprend une séquence d'acides aminés choisie dans le groupe constitué de 
Gly-Gly-Arg-Gly-Asp-Ser-Gly-Pro-Pro
Gly-Pro-Pro-Gly-Gly-Arg-Gly-Asp-Ser
Gly-Tyr-Ile-Gly-Ser-Arg-Gly-Pro-Pro;
 et

Gly-Pro-Pro-Gly-Tyr-Ile-Gly-Ser-Arg.

10. Copolymère bloc analogue au collagène selon la revendication 1, où au moins 50% environ des acides aminés, x et y, dans l'unité bloc C sont chacun indépendamment choisis dans le groupe constitué de la proline, de l'hydroxyproline, de la glycine, de l'alanine, et de la sérine.

11. Copolymère bloc analogue au collagène selon la revendication 10, où au moins un des acides aminés restants, x et y, est indépendamment choisi dans le groupe constitué de l'acide aspartique, de l'acide glutamique, de la lysine, de l'arginine, de l'histidine, de la tyrosine, et de la cystéine.

12. Copolymère bloc analogue au collagène selon la revendication 10, où au moins un des acides aminés restants, x et y, est indépendamment choisi dans le groupe constitué de la lysine, de la cystéine, de la tyrosine, de l'acide aspartique, et de l'acide glutamique.

13. Copolymère bloc analogue au collagène selon la revendication 10, où au moins un des acides aminés restants, x et y, est indépendamment choisi dans le groupe constitué de l'acide aspartique, de l'acide glutamique, de la lysine, de l'arginine, de l'histidine, de la tyrosine, et de la cystéine et au moins un des acides aminés restants, x et y, est indépendamment choisi dans le groupe constitué de la lysine, de la cystéine, de la tyrosine, de l'acide aspartique, et de l'acide glutamique.

14. Copolymère bloc analogue au collagène selon la revendication 1, où l'unité bloc C comprend la séquence d'acides aminés:

Gly-Ala-His-Gly-Pro-Ala-Gly-Pro-Lys.

15. Copolymère bloc analogue au collagène selon la revendication 1, où l'unité bloc C comprend une séquence d'acides aminés choisie dans le groupe constitué de: 
Gly-Pro-Lys-Gly-Lys-Ala-Gly-Pro-Lys
Gly-Pro-His-Gly-Lys-Ala-Gly-Pro-Lys
Gly-Pro-His-Gly-Lys-Ala-Gly-Pro-His
Gly-Pro-His-Gly-His-Ala-Gly-Pro-His
Gly-Pro-Glu-Gly-His-Ala-Gly-Pro-Lys
Gly-Pro-Glu-Gly-His-Ala-Gly-Pro-His
Gly-Pro-Glu-Gly-His-Ala-Gly-Pro-Glu;
 et

Gly-Pro-Glu-Gly-Glu-Ala-Gly-Pro-Glu.

16. Copolymère bloc analogue au collagène selon la revendication 1, répondant à la formule:
[C₂ A₂₄ C₂]₁₋₈
où,
l'unité bloc A comprend la séquence d'acides aminés, Gly-Ala-Pro-Gly-Pro-Ala-Gly-Pro-Pro, et
l'unité bloc C comprend la séquence d'acides aminés,

Gly-Ala-His-Gly-Pro-Ala-Gly-Pro-Lys.

17. Copolymère bloc analogue au collagène selon la revendication 16, répondant à la formule:
[C₂ A₂₄ C₂]₄.

18. Copolymère bloc analogue au collagène selon la revendication 1, répondant à la formule:
[C₂ A₁₂ C₂]₂₋₁₆
où,
l'unité bloc A comprend la séquence d'acides aminés, Gly-Ala-Pro-Gly-Pro-Ala-Gly-Pro-Pro, et
l'unité bloc C comprend la séquence d'acides aminés,

Gly-Ala-His-Gly-Pro-Ala-Gly-Pro-Lys.

19. Copolymère bloc analogue au collagène selon la revendication 18, répondant à la formule:
[C₂ A₁₂ C₂]₈.

20. Copolymère bloc analogue au collagène selon la revendication 1, répondant à la formule:
[C₂ (A B)₁₂ C₂]₁₋₈
où,
l'unité bloc A comprend la séquence d'acides aminés, Gly-Ala-Pro-Gly-Pro-Ala-Gly-Pro-Pro,
l'unité bloc B comprend la séquence d'acides aminés, Gly-Ser-Arg-Gly-Asp-Pro-Gly-Pro-Pro, et
l'unité bloc C comprend la séquence d'acides aminés,

Gly-Ala-His-Gly-Pro-Ala-Gly-Pro-Lys.

21. Copolymère bloc analogue au collagène selon la revendication 20, répondant à la formule:
[C₂ (A B)₁₂ C₂]₄.

22. Copolymère bloc analogue au collagène selon la revendication 1, répondant à la formule:
[C₂ B₁₂ C₂]₁₋₈
où,
l'unité bloc B comprend la séquence d'acides aminés, 
Gly-Ala-Gln-Gly-Pro-Ala-Gly-Pro-Gly-
Gly-Ser-Arg-Gly-Asp-Pro-Gly-Pro-Pro;
 et
l'unité bloc C comprend la séquence d'acides aminés,

Gly-Ala-His-Gly-Pro-Ala-Gly-Pro-Lys.

23. Copolymère bloc analogue au collagène selon la revendication 22, répondant à la formule:
[C₂ B₁₂ C₂]₄.

24. Copolymère bloc analogue au collagène selon la revendication 1, répondant à la formule:
[C₂ B₆ C₂]₂₋₁₆
où,
l'unité bloc B comprend la séquence d'acides aminés, 
Gly-Ala-Gln-Gly-Pro-Ala-Gly-Pro-Gly-
Gly-Ser-Arg-Gly-Asp-Pro-Gly-Pro-Pro;
 et
l'unité bloc C comprend la séquence d'acides aminés,

Gly-Ala-His-Gly-Pro-Ala-Gly-Pro-Lys.

25. Copolymère bloc analogue au collagène selon la revendication 24, répondant à la formule:
[C₂ B₆ C₂]₈.

26. Copolymère bloc analogue au collagène selon la revendication 1, répondant à la formule:
[C₂ B₂₄ C₂]₁₋₈
où,
l'unité bloc B comprend la séquence d'acides aminés, Gly-Ala-Gln-Gly-Pro-Ala-Gly-Pro-Gly, et
l'unité bloc C comprend la séquence d'acides aminés,

Gly-Ala-His-Gly-Pro-Ala-Gly-Pro-Lys.

27. Copolymère bloc analogue au collagène selon la revendication 26, répondant à la formule:
[C₂ B₂₄ C₂]₄.

28. Mélange homogène polymérique comprenant le copolymère bloc analogue au collagène selon la revendication 1, 2 ou 3 et du collagène naturel.

29. Fibre comprenant le copolymère bloc analogue au collagène selon la revendication 1, 2 ou 3.

30. Film comprenant le copolymère bloc analogue au collagène selon la revendication 1, 2 ou 3.

31. Revêtement comprenant le copolymère bloc analogue au collagène selon la revendication 1, 2 ou 3.

32. Implant médical comprenant le copolymère bloc analogue au collagène selon la revendication 1, 2 ou 3.
